(19) Europäisches Patentamt
European Patent Office
Office européen des brevets

(11) **EP 2 584 956 B1**

(12) **EUROPÄISCHE PATENTSCHRIFT**

(45) Veröffentlichungstag und Bekanntmachung des Hinweises auf die Patenterteilung:
**04.08.2021   Patentblatt 2021/31**

(21) Anmeldenummer: **11726446.5**

(22) Anmeldetag: **21.06.2011**

(51) Int Cl.:
*A61B 5/154* (2006.01)   *G01J 3/18* (2006.01)
*G01J 3/42* (2006.01)   *G01N 21/31* (2006.01)
*A61B 5/1455* (2006.01)   *A61B 5/00* (2006.01)
*G01J 3/02* (2006.01)   *G01J 3/28* (2006.01)
*A61B 5/145* (2006.01)

(86) Internationale Anmeldenummer:
**PCT/EP2011/060337**

(87) Internationale Veröffentlichungsnummer:
**WO 2011/161102 (29.12.2011 Gazette 2011/52)**

(54) **VORRICHTUNG UND VERFAHREN ZUM ERKENNEN UND ÜBERWACHEN VON PHYSIOLOGISCHEN BLUTWERTEN**

DEVICE AND METHOD FOR RECOGNISING AND MONITORING PHYSIOLOGICAL BLOOD VALUES

DISPOSITIF ET PROCÉDÉ DE RECONNAISSANCE ET DE SURVEILLANCE DE VALEURS SANGUINES PHYSIOLOGIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.06.2010   EP 10166854**
**21.04.2011   EP 11163361**

(43) Veröffentlichungstag der Anmeldung:
**01.05.2013   Patentblatt 2013/18**

(73) Patentinhaber: Sentec GmbH
**18069 Rostock (DE)**

(72) Erfinder: **KULCKE, Axel**
**A-8382 Weichselbaum (AT)**

(74) Vertreter: **Hepp Wenger Ryffel AG**
**Friedtalweg 5**
**9500 Wil (CH)**

(56) Entgegenhaltungen:
WO-A1-93/16629          WO-A1-2008/116835
WO-A1-2009/139315     US-A- 5 159 199
US-A- 5 361 758          US-A- 6 049 727
US-A- 6 078 828          US-A1- 2003 071 216
US-A1- 2005 154 277     US-A1- 2005 267 346
US-A1- 2007 216 898     US-A1- 2008 208 020
US-A1- 2010 046 826     US-B1- 6 315 955

**Beschreibung**

Die Erfindung betrifft eine Vorrichtung und ein Verfahren zum Erkennen und Überwachen von physiologischen Blutwerten eines Lebewesens, mit den Merkmalen der unabhängigen Patentansprüche. Die Überwachung und Messung von Inhaltsstoffen oder Eigenschaften eines Messmediums wird heute in einer Vielzahl von medizinischen und nichtmedizinischen Anwendungen vorgenommen. Beispielsweise sollen Inhaltsstoffe im Blut eines Patienten (z. B. Blutzucker oder Sauerstoffsättigung) oder in Prozessfluiden (Flüssigkeiten oder Gasen) von nichtmedizinischen Anwendungen (z. B. Prozessüberwachungen) online erkannt und kontinuierlich überwacht werden.

Pulsoximeterie ist beispielsweise ein Verfahren, welches die Pulsfrequenz (PR = pulse rate) und die prozentuelle Sauerstoffsättigung (%SpO2) des arteriellen Blutes bestimmt. Dieses Verfahren ist heute etabliert und wird in vielen Bereichen der Medizin eingesetzt, zum Beispiel in der Intensivmedizin, zum Schlafmonitoring und während Operationen.

Bei der Grundtechnologie ist es üblich, zwei Wellenlängen (typisch 660 nm und 940nm), die durch LEDs erzeugt werden, zeitlich schnell getaktet mit einem optischen Sensor aufzunehmen. Durch unterschiedliche Signalintensitäten im variablen und fixen Bereich lassen sich die gewünschten Messwerte extrahieren. Durch die Transparenz des Gewebes in dem betrachteten Spektralbereich können bei starkem und vor allem variierendem Aussenlicht durch Änderungen Zusatzsignale erzeugt werden. Diese werden im Allgemeinen jeweils durch einen dritten Messpunkt ohne LED-Beleuchtung abgefangen.

Ein allgemeines Problem besteht darin, dass bei optischen Messtechniken die in das Gewebe einleitbare Lichtmenge beschränkt ist. Ansonsten ist aufgrund von thermischen Effekten mit einer Beschädigung des Gewebes zu rechnen. Aus diesem Grund sind bei bekannten Messverfahren oder Vorrichtungen die Messzeiten verhältnismässig lang. Dies führt zu einem schlechten Signal-/Rauschverhältnis. Gerade bei der Messung von Stoffen, welche in sehr kleinen Konzentrationen vorliegen (beispielsweise bei Blutzuckermessungen, wo Konzentrationen im Bereich von mmol/l gemessen werden), ist ein möglichst gutes Signal-/Rauschverhältnis aber zwingend erforderlich.

Heutzutage ist es gewünscht, neben der Sauerstoffsättigung des Hämoglobins weitere Blutparameter zu erfassen, um wichtige Parameter bei der Patientenüberwachung zu erhalten.

Zum einen ist es wichtig, den Gashaushalt im Blut zu überwachen. In WO 2008/132205 A1 ist ein Sensor beschrieben, mit dem neben der Pulsoximetrie der CO2-Partialdruck im Gewebe bestimmt werden kann.

Weitere wichtige Werte zur Überwachung sind die unterschiedlichen Hämoglobinderivate. Dazu gehören die Bestimmung der gesamten Hämoglobinkonzentration (ctHb), der Bestimmung der Carboxyhämoglobinkonzentration (HbCO) oder anderer Blutwerte. Diese zusätzlichen Blutwerte können allerdings mit der vorstehend beschriebenen Technologie (2 Wellenlängen) nicht erfasst werden. Voraussetzung für die Erfassung ist die sehr präzise Bestimmung der spektralen Eigenschaften des arteriellen Blutes und des Gewebes über einen großen Spektralbereich.

Ein anderer zu messender Blutwert ist der Blutzuckergehalt. Diabetes mellitus ist eine der weltweit häufigsten Stoffwechselerkrankungen und hat eine durch die sich ändernden Ernährungsgewohnheiten stark zunehmende Tendenz. So geht man im Jahr 2010 von ca. 285 Mio. Personen (6,4% der Weltbevölkerung) aus und 2030 von bereits 439 Mio. Personen (7,7% der Bevölkerung). Dies ist einer der Gründe dafür, dass die Glucosekonzentration in Körperflüssigkeiten einer der am meisten bestimmten Parameter in der klinischen Chemie ist. Idealerweise möchte man hier den genauen Blutzuckerspiegel mit nichtinvasiven Methoden bestimmen.

Bei der Patientenüberwachung wurde zur Untersuchung zusätzlicher Blutparameter in WO 2006/094169 A1 ein LED-basiertes, photometrisches System vorgeschlagen, welches eine Vielzahl (typisch acht) LEDs unterschiedlicher Wellenlängen hat und somit an unterschiedlichen spektralen Stützstellen Signale erheben kann. Diese Technologie weist aber mehrere Nachteile auf. Die LEDs werden sequentiell geschaltet und mit einem breitbandigen Sensor zeitlich versetzt aufgenommen und haben so bei variierenden externen Lichteinflüssen und Bewegungen am Sensor häufig virtuelle überlagerte Signale. Da bei dieser Technologie viele LEDs und somit Stützstellen sequentiell geschaltet werden müssen und die Auswertung vorwiegend aus der relativen Intensität der Signale zueinander durchgeführt werden muss, wirken sich solche Überlagerungen besonders stark aus. Weiterhin haben LEDs eine temperaturabhängige Abstrahlungskurve, und die Abstrahlung der LEDS ist üblicherweise mit spektralen Halbwertsbreiten zwischen 20 nm und 30 nm unterlegt, was keine präzise Eingrenzung auf einen engen Spektralbereich und somit auf eine relevante chemische Komponente zulässt. Ausserdem ist durch die geringe Zahl an Stützstellen in Kombination mit von Mensch zu Mensch variierenden Eigenschaften der Hautoberfläche und des Gewebes eine präzise Separation und quantitative Auswertung der Blutkomponenten nur begrenzt möglich. Aus der Messung können nur Verhältnisse von wenigen Stützpunkten generiert und zur Analyse der chemischen Komponenten eingesetzt werden. Da sich die Spektroskopie des Blutes und vor allem die Eigenschaften des mit Licht zu durchdringenden Gewebes (unterschiedliche Streueigenschaften von Person zu Person und auch abhängig von der Wellenlänge) aber stark auf die Messergebnisse auswirken, ist die Messung mit dieser Methode fehleranfällig.

Vielseitige Entwicklungen ermöglichen heutzutage eine zuverlässige Glucosekonzentrationsbestimmung in Blut und hiervon abgeleiteten Flüssigkeiten wie Blutplasma und Serum, aber auch in anderen Körperflüssigkeiten wie beispielsweise Urin. In den letzten Jahren haben sich insbesondere unterschiedliche enzymatische Methoden etablieren können. Dabei verlangt die Überzahl der Methoden aber eine geringe Blutabnahme und gehört so zu den invasiven Methoden.

Die gegenwärtige Praxis der Glucosemessungen reicht von selten - beim Arztbesuch - bis hin zu mehrfach pro Stunde gemessenen Werten bei Patienten auf der Intensivstation. Bei insulinpflichtigen Diabetikern ist eine bis zu sechsmal täglich erfolgende Selbstkontrolle mit Hilfe von Teststreifengeräten üblich, um eine verbesserte, jedoch nicht optimale Regulation der Blutglucosekonzentration zu erreichen. Solche Messungen erfordern eine Blutentnahme, welche für den Patienten unangenehm ist. Ausserdem ist das Blut aufgrund des Rausdrückens nicht im Gleichgewicht mit der Gewebeflüssigkeit. Dies führt zu Ungenauigkeiten.

Daher wäre die Umsetzung eines nichtinvasiven Verfahrens und einer dazugehörigen Vorrichtung für die Medizintechnik sehr wünschenswert.

Zur Blutzuckerbestimmung muss quantitativ die Glucosekonzentration im Blut bestimmt werden.

Es wurden bereits spektroskopische Verfahren entwickelt, die eine reagenzfreie Bestimmung der Glucose in komplizierten Körperflüssigkeiten erlauben. Beispielsweise sind spektroskopische Messungen im Nahinfrarotbereich (NIR-Spektroskopie) bekannt. Dabei müssen geringe Konzentrationen an Glucose (ca. 2 mmol/l bis ca. 30 mmol/l; Zielbereich 5,0 bis 7,0 mmol/1) nachgewiesen werden. Weiterhin ist im Blut ein sehr hoher Wasseranteil (typisch größer 80%) vorhanden, der somit viel stärkere Absorptionen in der NIR Spektroskopie hervorruft. Weiterhin enthält das Blut andere Substanzen in variierenden und unbekannten Konzentrationen, und es muss eine Querempfindlichkeit ausgeschlossen werden. Wenn nichtinvasiv gemessen werden soll, muss im Körper innerhalb des umliegenden Gewebes gemessen werden, und die Einflüsse der unterschiedlichen Medien sind zu berücksichtigen oder zu separieren.

Weiterhin muss man bei einer nichtinvasiven Messmethode eine Messstelle am Körper auswählen. Vorteilhafte Messstellen haben nicht immer das Blut separat vorliegen, sondern das Blut in Gewebe eingelagert. Ausserdem ist das Gewebe von einer Hautschicht überzogen, bei der von Person zu Person unterschiedliche Eigenschaften vorliegen, die sich auch zeitlich stark ändern können. So ist zum Beispiel der Wassergehalt der Haut stark fluktuierend und hängt unter anderem von der Aktivität der Schweißbildung ab. Weiterhin hat die Haut unterschiedliche Aufbauten und kann an vielen Stellen eine subkutane Fettschicht besitzen, die zum einen durch einen sehr viel niedrigeren Wassergehalt gekennzeichnet ist, zum anderen auch eine deutlich niedrigere Durchblutung hat und somit insbesondere zum Blutzucker nicht im Gleichgewicht stehen muss. Weiterhin ist häufig zu berücksichtigen, dass auch Knochengewebe im optischen Messbereich liegt. Es ist hier nicht selbstverständlich, dass das Knochengewebe mit den Inhaltsstoffen im Gleichgewicht ist.

Die wellenlängenabhängige Untersuchung von chemischen Komponenten kann auf unterschiedliche Art und Weise erfolgen. Die Messung kann an diskreten Stützstellen durchgeführt werden. Dies wird im Allgemeinen als photometrische Messtechnik oder multispektrale Photometrie bezeichnet und entweder mit mehreren Lichtsendern mit unterschiedlichen Wellenlängen durchgeführt oder es wird breitbandiges "weißes Licht" eingesetzt und es kommen mehrere spektral eingeschränkte Empfänger zum Einsatz (Filtertechnologie). Diese Lichtsender mit unterschiedlichen Wellenlängen können mit LEDs oder mit Lasern und mit breitbandigen Beleuchtungen und schmalbandigen Filtern über den Photoempfängern realisiert sein. Bei dem Einsatz von LEDs kommt technisch erschwerend hinzu, dass sie zum einen relativ große Strahlungsverteilungen haben und zum anderen sich die Strahlungsverteilung beim Erwärmen der Emitter verändert. Ein Beispiel dieser Technologie wurde beispielsweise in US 5,086,229 beschrieben.

Diese Messtechniken haben typisch drei bis ca. zehn Wellenlängen oder Wellenlängenbereiche, die aufgenommen und ausgewertet werden.

Auch diese Messtechnik ist für die Anwendung zur Blutzuckerbestimmung nicht zielführend. Zum einen müssen mit dieser Messtechnik im effektiven Wellenlängenbereich zwischen 800 nm und 1200 nm mehrere Stützpunkte realisiert werden. Dabei müssen diese Stützpunkte drei Anforderungen erfüllen:

1. Sie müssen auf und ausserhalb von Absorptionsbanden der Glukose und des Wassers liegen.
2. Sie müssen unabhängig von Querempfindlichkeiten zu anderen Substanzen sein, die im Blut oder Gewebe vorkommen können.
3. Sie müssen so eingerichtet sein, das man die unterschiedlichen Streusignale und Lichtwege und somit die unterschiedlichen Grundsignale herausrechnen kann.

Für eine nichtinvasive Messung des Blutzuckergehaltes müssen zusammenfassend also die folgenden Bedingungen erfüllt sein: eine spektroskopische Erkennung von kleinen Glucosekonzentrationen im Vergleich zu Wasserkonzentrationen muss möglich sein. Eine tiefe Durchdringung durch das Gewebe (typisch größer 3 mm) ist notwendig. Die Querempfindlichkeit zu anderen Substanzen muss ausgeschlossen werden können. Werte aus Blut und Gewebe müssen unterschieden werden können (pulsatiler Check). Die Geräte müssen günstig und klein und damit portabel sein. Die Lichtquelle darf nicht zu stark sein, so dass keine Verbrennungen im Finger entstehen, bevorzugt sollte die Lichtquelle auf der Basis von LEDs sein.

Bis heute konnte kein Seriengerät entwickelt werden, welches eine nichtinvasive regelmässige, sichere und zuverlässige Blutzuckerbestimmung für einen Diabetiker ermöglicht. Viele der bisher vorgeschlagenen Methoden und Vorrichtungen sind nicht geeignet, diese Probleme zu lösen oder die vorstehenden Voraussetzungen zu erfüllen.

Ein nichtinvasiver Sensor zur Blutzuckermessung ist z. B. aus US 5,070,874 und US 5,360,004 bekannt.

Es sind ausserdem reflexive Messtechniken allgemein bekannt. Diese sind für die Blutzuckermessung nur bedingt bis gar nicht einsetzbar. Zum einen kommt der vorwiegende Teil der Strahlung direkt von der Oberfläche, zum anderen hat die Haut einen von Person zu Person und auch von Körperstelle zu Körperstelle unterschiedlichen Schichtaufbau. Weiterhin können die Streueigenschaften nicht sicher definiert werden. Somit ist eine stabile quantitative Messung in der geforderten niedrigen Substanzkonzentration bei der geforderten Aufgabenstellung mit reflexionsspektroskopischen Methoden nicht zielführend.

Die Spektroskopie wird für die Konzentrationsbestimmung organischer Materialien eingesetzt und ist in der medizinischen Forschung als Grundtechnologie häufig im Einsatz. Dabei ist es üblich, Blut in geringen Mengen abzunehmen und dieses photometrisch oder spektroskopisch in vitro zu untersuchen. Der Geräte- und Durchführungsaufwand für diese invasive Technologie ist erheblich. Weiterhin ist sie zeitverzögert. Eine In-vitro-Bestimmung ist für die Patientenüberwachung (Monitoring) nur sehr bedingt einsetzbar.

Bei der spektroskopischen Messtechnik findet eine Zerlegung des Lichtes über einen breiten Spektralbereich mit einem Spektrometer statt, wobei im Allgemeinen heute eine Gitterstruktur eingesetzt wird und das Licht spektral auf einen Sensor mit vielen in einer Linie angeordneten Photoempfängern (Pixeln) aufgenommen und analysiert wird. Eine weitere Möglichkeit ist das Fourier-Transform-Verfahren (FTIR-Spektroskopie), welches bevorzugt im nahen Infrarot eingesetzt wird.

Auch diese Methode ist für die Blutzuckerbestimmung nicht ideal. Zum einen ist diese Methode besser für längerwellige Strahlung geeignet. Zum anderen ist sie durch das Fourierprinzip zwar gut für schmalbandige Peaks geeignet. Bei breitbandigen Absorptionen, welche im Bereich 800 nm bis 1200 nm für die quantitative Analyse von Wasser und Glucose im Blut benötigt wird, ist sie aber eine eher unpräzise und störungsanfällige Messmethode.

Die Erfinder haben erkannt, dass für Blutzuckermessungen nur Methoden im Spektralbereich zwischen 650 nm und ca. 1200 nm zum Einsatz kommen sollten, da ansonsten die mögliche Weglänge im Gewebe zu kurz ist und die Einflüsse der Haut und äußeren Schichten zu groß sind. Dieser Spektralbereich ist in der Medizintechnik als diagnostisches Fenster bekannt. Der Bereich ermöglicht ein Analysieren der Substanzen auch unterhalb der Hautoberfläche. Es ist weiterhin bekannt, dass durch die starken Streueigenschaften des Gewebes die effektive Weglänge des Lichtes stark von der direkten Weglänge abweicht. Eine typische Größe für die Abschätzung ist Faktor 4 bis Faktor 8 für die effektive Weglänge zur direkten Weglänge.

Da die Mehrzahl der bekannten Methoden zur Blutzuckermessung aber bei Wellenlängen oberhalb von 1300 nm messen, da hier stärkere Signale der Absorptionsbanden auftreten, bleiben wenige der bekannten Methoden zur Blutzuckerbestimmung übrig.

Weitere Ansätze für die Blutzuckerbestimmung wurden von Fischbacher et al. (Ch. Fischbacher, K.-U. Jagemann, K. Danzer, U. A. Müller, L. Papenkordt, J. Schüler; Enhancing calibration models for non-invasive near-infrared spectroscopical blood glucose determination; Fresenius J Anal Chem (1997) 359: 78-82 Springer-Verlag 1997) und von Meuer et al. (Non-invasive glucose determination in the human eye; Wolfgang Schrader, Petra Meuer, Jürgen Popp, Wolfgang Kiefer, Johannes-Ulrich Menzebach and Bernhard; SchraderJournal of Molecular Structure, Volumes 735-736, 14 February 2005, Pages 299-306 sowie Dissertation Petra Meuer, Universität Würzburg 2002) vorgeschlagen.

Fischbacher et al. zeigen, dass eine enge Verknüpfung hergestellt werden kann. Das Signal-/Rauschverhältnis von üblichen Spektroskopiegeräten wurde jedoch als nicht ausreichend erkannt. Weiterhin wurde in Reflexion gemessen, was im Gewebe wie oben beschrieben nicht zielführend ist.

Meuer et al. zeigen bei den Messungen am Augapfel auch gute Ergebnisse. Das vorgeschlagene Verfahren kann aufgrund des klaren, nicht streuenden Mediums auch in Reflexion eingesetzt werden. Aber auch hier zeigte sich deutlich, dass kommerziell angebotene Spektroskopietechniken das notwendige Signal-/Rauschverhältnis für eine sichere Bestimmung der niedrigen Konzentrationen nicht erfüllen.

Weitere Anwendungsgebiete, in denen zeitaufgelöst Inhaltsstoffe eines Messmediums ermittelt werden müssen, betreffen die Messung von Laktat im Blut, die Dialyse oder das Blut in einer Herz-Lungen-Maschine, wo ebenfalls Blutwerte (jedoch gegebenenfalls in vitro) ermittelt werden oder auch nichtmedizinische Anwendungen, wo z. B. Prozessfluide überwacht werden sollen. Typische nichtmedizinische Anwendungen sind beispielsweise Farbmessungen von Flüssigkeiten in Produktionsprozessen. Ebenfalls ist es denkbar, Gase in Verbrennungsprozessen zu messen. Weitere Anwendungsmöglichkeiten ergeben sich beispielsweise in der Lebensmitteltechnologie bei der Zudosierung von Inhaltsstoffen, welche kontinuierlich gefördert werden.

Es ist bereits bekannt, Laborspektrometer für Untersuchungen von Blut und Gewebekomponenten in vivo einzusetzen. Moderne Laborspektrometer arbeiten dem Stand der Technik entsprechend heute mit Zeilensensoren. Laborspektrometer arbeiten üblicherweise mit Glasfaseranschlüssen, und so müssten aufwendige Lichtleiterlösungen für die Beleuchtung und die Lichterfassung von der Messstelle (Sensor am Gewebe) bis zu einer Geräteeinheit mit Spektrometer geführt werden. Bei gleichzeitigen doch erheblichen Lichtverlusten (gerade bei der Lichteinkopplung) ist die zeitaufgelöste Messung nur mit schlechten Signal-/Rauschverhältnissen realisierbar.

Daher ist die Anwendung von solchen Laborspektrometern für die Überwachung oder Messung von Blutwerten wie z. B. der Sauerstoffsättigung oder der Blutzuckerbestimmung ebenfalls nicht zielführend.

Monochromatorsysteme und FTIR-Spektrometer erfüllen nicht die Zeiterfordernisse an eine kombinierte Spektralanalyse und Puls-überwachung.

Mit einer Glasfaser kann nur ein geringer Anteil des effektiven Lichtes aufgefangen werden. Die Einheiten benötigen daher im Allgemeinen lange Integrationszeiten bei der Messung. Aus EP 522 674 A2 ist ein Oxymeter zur Bestimmung der Blutsauerstoffsättigung in einem Fötus bekannt. Dazu wird ein Spektrometer verwendet, in welches Messlicht von einer Messstelle mittels Glasfasern zu einem Spektrometer übermittelt wird.

Aus US 2006/0167348 ist es bekannt, in vivo Infrarotspektren mittels eines konventionellen FTIR-Spektrometers zu erstellen. Auch dazu wird eine Glasfaserübertragung des Messlichts vorgeschlagen.

Aus WO 2009/043554 ist ein Verfahren und eine Messeinrichtung zur Erhebung spektrometischer Messsignale aus vitalem Gewebe bekannt. Die Art und Weise, wie das Messlicht in die Sensoranordnung eingekoppelt wird, ist allerdings hier nicht gezeigt. Durch die notwendige Lichtzerlegung der Spektrometer und die begrenzte Lichtintensität, mit der die Hautoberfläche des Körpers belastet werden kann, konnten diese Messungen deshalb bisher nicht zeitaufgelöst, das heißt pulsaufgelöst, durchgeführt werden. Dies wäre aber notwendig, um zwischen pulsatilem Anteil und Gewebeanteil der gemessenen Werte zu unterscheiden.

Weiterhin dürfen die Sensoren oder zu mindestens die Teile, die am Körper angebracht werden, eine bestimmte Größe nicht überschreiten, so dass sie in der Praxis bei Patienten bei einer Langzeitüberwachung nicht störend wirken.

Ein System zur spektralen photometrischen Messung mit zeitaufgelöster Messwertaufnahme ist in WO 03/071939 A1 vorgeschlagen. Hierbei wird eine breitbandige Lichtquelle mit unterschiedlichen spektralen Filtern sequentiell gemessen. Dieses System ist sehr groß und aufwendig. Die zeitaufgelösten Informationen werden ausserdem immer nur bei einer Wellenlänge aufgenommen und die Wellenlängen nacheinander aufgenommen. Daher ist dieses System, welches auch in einem anderen Spektralbereich und zur Überwachung des Blutzuckers eingesetzt werden sollte, nicht für die Langzeitüberwachung der Puls- und Blutparameter geeignet.

In US 5,879,294 ist ein System vorgeschlagen, bei dem spektroskopische Messungen von Chromophoren im Gewebe durchgeführt werden. Hierbei wird zur Auswertung die zweite Ableitung eines Spektrums eingesetzt und an Stützstellen (typisch zwei pro Substanz) die Auswertung durchgeführt. So kann zum Beispiel die Sauerstoffsättigung im Gewebe bestimmt werden. Mit diesem Verfahren kann die statische, also die nicht puls- oder zeitaufgelöste, quantitative Bestimmung der Chromophore durchgeführt werden. Ein daraus folgendes Verfahren zur Überwachung der Gewebesauerstoffkonzentration (StO2-Konzentration) ist in WO 2007/048989 A1 dargestellt.

Bei der Patientenüberwachung (beispielsweise zur Ermittlung der Sauerstoffsättigung) muss ausserdem eine Unterscheidung zwischen Anteilen im Blut (Hämoglobin) und Anteilen im Gewebe (Myoglobin) gemacht werden. Die spektralen Eigenschaften der Hämoglobin- und Myoglobin-Modifikationen sind sehr ähnlich, aber bei spektral hochauflösender Untersuchung unterschiedlich. Ein Verfahren, welches eine Unterscheidung erlaubt, ist in US 5,931,779 beschrieben.

Bei der Messung von Blutzucker ist eine Differenzierung zwischen Gewebe und Blut hingegen nicht zwingend erforderlich. Eine pulsaufgelöste Messung ist daher bei der Blutzuckermessung nicht zwingend notwendig. Es werden (wenn gut durchblutetes Gewebe ohne zum Beispiel subkutanes Fett und Knochen vorliegt) genaue Werte erreicht, wenn ein Gleichgewicht zwischen dem Blutzuckergehalt im Blut und im Gewebe besteht. Über den pulsatilen Anteil kann aber kontrolliert werden, ob ein Gleichgewicht vorliegt.

Eine weitere, schwierige Restriktion im Bereich der in vivo Blutanalyse ist die starke Abnahme der Absorption oder Extinktion der relevanten Blut-, Gewebe- und Hautbestanteile über den für die Patientenüberwachung medizinisch wichtigen Spektralbereich von 500 nm bis 850 nm. So haben im sichtbaren Spektralbereich das Hämoglobin und das in der Haut vorhandene Melanin sehr große Absorptionskoeffizienten, während im sehr nahen Infrarotbereich (VNIR) diese deutlich niedriger sind.

Bei der Messung von Blutzucker, welcher bevorzugt typischerweise in einem Wellenlängenbereich von 800 nm bis 1200 nm bestimmt wird, ist die Absorption im Gewebe geringer, so dass bei der Blutzuckermessung dieses Problem weniger ins Gewicht fällt.

Eine vergleichbare Problematik kann sich aber auch bei der Invitro-Messung von Blutwerten z. B. bei Dialysepatienten oder bei der nichtmedizinischen Messung und Überwachung von Prozessparametern an schwierig zugänglichen Maschinenteilen, beispielsweise in Rohrleitungen, ergeben. Auch bei solchen Anwendungen ist eine zeitaufgelöste Messung erforderlich, und Laborspektrometer mit grossen Dimensionen können nicht ohne weiteres an die Messstelle gebracht werden.

Die US 5 361 758 beschreibt eine Vorrichtung zum Erkennen und Überwachen von Blutkonzentrationen oder Gewebeeinhaltsstoffen. Breitbandiges Licht wird von einer Messstelle zurückgeworfen, aufgefächert und auf einen linearen Detektor geleitet, welcher aus einzelnen Dioden besteht.

Aus der US2007/216898 A1 ist eine Vorrichtung zum Erkennen von Inhaltsstoffen eines Messmediums, insbesondere von schädlichen Stoffen in der Umwelt, bekannt. Die Vorrichtung umfasst eine Lichtquelle, Mittel zum Auffächern des Analyselichtes und einen zweidimensionalen Sensor-Array, wobei das Analyselicht an unterschiedlichen Stellen des Sensor-Array auftrifft.

US 6 315 955 beschreibt eine Vorrichtung zur quantitativen Bestimmung von Partikeln in Flüssigkeiten. Die Vorrichtung umfasst eine Lichtquelle und einen Detektor.

US 2005/154277 A1 offenbart eine Kapsel zum Einführen in einen biologischen Körper mit einer Lichtquelle, einem Detektor und einem Mikrospektrometer.

US 6 049 727 zeigt eine implantierbare Vorrichtung zur Bestimmung von Konzentrationen von Bestandteilen von Körperflüssigkeiten. Die Vorrichtung umfasst eine Lichtquelle und einen Detektor.

WO 93/16629 A1 zeigt eine Vorrichtung zum kontinuierlichen Erfassen und Überwachen von Konzentrationen von Blutbestandteilen. Die Vorrichtung umfasst eine Lichtquelle, Mittel zum Auffächern des Analyselichtes sowie einen linearen Array Detektor.

US 2008/208020 A1 beschreibt eine implantierbare Vorrichtung zum Überwachung der Gewebedurchblutung. Die Vorrichtung umfasst eine Lichtquelle und einen Detektor. Es kann breitbandiges Licht und ein Detektor mit linearem Array verwendet werden.

US 2005/267346 A1 offenbart eine Vorrichtung zur Bestimmung von Blutbestandteilen. Sie umfasst eine Lichtquelle und einen Detektor. Bei Verwendung von breitbandigem Licht kann der Lichtquelle ein Filter nachgeschaltet werden, um für die Messung nur Licht einer bestimmten Wellenlänge zur Verfügung zu stellen.

US 6 078 828 beschreibt ein optisches Messsystem. Das Messsystem beinhaltet eine Lichtquelle, ein Spektrometer und einen Detektor.

US 2003/071216 A1 beschreibt eine Vorrichtung zum Bestimmen des IR-Spektrums einer Probe. Die Vorrichtung umfasst eine Lichtquelle, Mittel zum Auffächern des zurückgeworfenen Analyselichtes sowie einen zweidimensionalen Detektor. Die Messvorrichtung ist im Zusammenhang mit optischem Fasern einsetzbar, um eine medizinische Endoskopie durchzuführen.

WO 2009/139315 A1 zeigt ein spektroskopisches Modul. Das Modul umfasst eine Linseneinheit, in welcher einfallendes Licht in eine Vielzahl von Wellenlängen aufgefächert wird, und ein Sensorelement zum Erfassen der Intensitäten der spezifischen Wellenlängen. Der Lichtsensor kann ein zweidimensionales Pixelfeld umfassen, wobei eine Linie von Pixeln in einem Kanal aufgesammelt wird.

Alle bekannten Lösungen sind also mit Nachteilen behaftet. Es gibt insbesondere kein Spektroskopiesystem, welches alle Anforderungen an eine Pulsmessung, an eine in vivo Blutanalyse mit Unterscheidung nach pulsatilem Anteil (arterielles Blut) und statischem Anteil (venöses Blut und Gewebe, Myoglobin) und eine Miniaturisierung der Sensoreinheit zum kontinuierlichen Einsatz am Patienten, an eine nichtinvasive Blutzuckermessung oder an die Messung von Inhaltsstoffen eines Messmediums an Messstellen mit beschränktem Platz gemeinsam erfüllt.

Diese vorstehend zusammengefassten Restriktionen schienen es bis heute unmöglich zu machen, Untersuchungen in vivo und zeitaufgelöst und/oder mit einer Vorrichtung, die einen geringen Platzbedarf hat und robust ist, durchzuführen.

Es ist deshalb eine Aufgabe der vorliegenden Erfindung, die Nachteile des Bekannten zu vermeiden und insbesondere eine Vorrichtung und ein Verfahren zu schaffen, welche die vorstehend aufgeführten Restriktionen nicht aufweisen und welche es insbesondere erlauben, die gewünschten Analysen in vivo und zeitaufgelöst, das heißt mit der physiologischen Unterscheidung von arteriellen Blutparametern und gewebeabhängigen Parametern, durchzuführen. Ausserdem soll es ermöglicht werden, zeitaufgelöste Messungen von Blutzucker in vivo und Blutmessungen in vitro oder auch zeitaufgelöste Messungen in nichtmedizinischen Anwendungsbereichen zuverlässig und auch an schlecht zugänglichen Messstellen durchführen zu können.

Erfindungsgemäss werden diese und weitere Aufgaben mit einer Vorrichtung und einem Verfahren mit den Merkmalen der unabhängigen Patentansprüche gelöst.

Die theoretische Grundlage für die spektroskopischen oder photometrischen Untersuchungen ist das Beer-Lambertsche Gesetz. Mit diesem können Konzentrationen $c_i$ von absorbierenden Molekülen in Lösungen bei dem Durchtritt von Licht bestimmt werden.

$$I_\lambda = I_{0,\lambda}\, e^{-\mu_{a\lambda} l_\lambda} \qquad (1)$$

mit $I_\lambda$ der Lichtintensität nach Durchtritt durch die zu untersuchenden Substanz, $I_{0,\lambda}$ der eingestrahlten Lichtintensität, $\mu_{\alpha,\lambda}$ dem wellenlängenabhängigen ($\lambda$) Gesamtabsorptionskoeffizienten und der Weglänge I durch die Substanz. Durch die streuenden Eigenschaften von Gewebe ist hier mit einer effektiven Weglänge zu rechnen, welche im Allgemeinen auch wellenlängenabhängig ist, was in diesem Spektralbereich und Anwendungsfall allerdings vernachlässigt werden kann.

Durch Umformung erhält man

$$\ln\left(\frac{I_\lambda}{I_{0,\lambda}}\right) = -l \cdot \mu_{a,\lambda} \qquad (2)$$

Dieses allgemeine Gesetz muss nun weiter diversifiziert werden, da eine Substanz wie zum Beispiel menschliches Blut aus vielen chemischen Teilsubstanzen (molekularen Verbindungen) besteht und deren Absorptionskoeffizienten sich wellenlängenabhängig unterscheiden. Bei n Substanzen erhält man

$$\mu_{a,\lambda} = \sum_{i=1}^{n} \varepsilon_{i,\lambda} \cdot c_i \qquad (3)$$

Für m Wellenlängen kann dies nun mit der Annahme, dass die Weglängen für alle Wellenlängen gleich bleiben, wie folgt umgeformt werden:

$$\begin{bmatrix} \ln\left(\frac{I_{\lambda 1}}{I_{0,\lambda 1}}\right) \\ \vdots \\ \ln\left(\frac{I_{\lambda m}}{I_{0,\lambda m}}\right) \end{bmatrix} = -l \begin{bmatrix} \varepsilon_{1,\lambda 1} & \cdots & \varepsilon_{n,\lambda 1} \\ \vdots & \ddots & \vdots \\ \varepsilon_{1,\lambda m} & \cdots & \varepsilon_{n,\lambda m} \end{bmatrix} \begin{bmatrix} c_1 \\ \vdots \\ c_n \end{bmatrix} \qquad (4)$$

Diese Beziehung kann nun wieder in folgender Form geschrieben werden:

$$\mathbf{I}(\lambda) = -l\,\mathbf{A}(\lambda)\mathbf{C} \qquad (5)$$

oder

$$C = -\frac{1}{l} A(\lambda)^{-1} I(\lambda) \qquad (6)$$

Daraus lassen sich die Konzentrationen der Substanzen direkt bestimmen.

Eine zusätzliche Grundlage ist die quantenchemische Wechselwirkung von Licht und Molekülen. So werden durch die wellenlängenabhängige Absorption von Lichtquanten diskrete und molekülspezifische Rotationsschwingungsübergänge oder elektronische Übergänge angeregt. Dabei finden die Anregungen im betrachteten Spektralbereich durch die Rotationsschwingungsanregung von Oberton- und Kombinationsschwingungen der Moleküle oder durch komplexe elektronische Übergänge in den Chromophoren statt. Diese Übergänge sind wellenlängenspezifisch und substanzspezifisch. So können bei unterschiedlichen Wellenlängen die unterschiedlichen Substanzen analysiert werden. Da z. B. im menschlichen Körper aber sehr viele unterschiedliche Substanzen vorliegen und sich die Informationen überlagern, ist es zur Berücksichtigung der quantenmechanischem Wechselwirkungen notwendig, die spektroskopische Methode einzusetzen und nicht die stützstellenbasierte, multispektrale Photometrie oder photometrische Analyse.

Bei sehr detaillierter Betrachtung der Obertonspektroskopie der zwei Substanzen Wasser und Glucose im Spektralbereich 800 nm bis 1200 nm müssen z. B. weitere Details berücksichtigt werden. Wasser ist hier ein sehr spezielles Molekül. Dies kommt zum einen von der sehr starken Polarität des Wassers mit der zusätzlich gewinkelten Anordnung der Atome. Weiterhin hat im flüssigen Zustand die Wasserstoffbrückenbindung einen Einfluss auf die Spektren. So ist auf der einen Seite die Spektroskopie am flüssigen Wasser stark temperaturabhängig. Dies kann hier aber vernachlässigt werden, da die Temperaturen an der Messstelle im Körper in einem engen Temperaturbereich von 35°C bis 40°C festgelegt sind. Einen weiteren Einfluss hat die Lösung der Moleküle in Wasser. So ändern sich bei Konzentrationen auch die Kräfte zwischen den Molekülen und die resultierenden Spektren. Diese Änderungen sind zwar relativ gering, aber nachweisbar und müssen in der chemometrischen Auswertung berücksichtigt werden.

Die erfindungsgemässe Vorrichtung dient zum Erkennen und Überwachen von physiologischen Blutwerten eines Lebewesens. Die Vorrichtung weist wenigstens eine Lichtquelle zum Erzeugen von breitbandigem Licht auf. Breitbandig bedeutet in diesem Zusammenhang, dass jedenfalls Licht mit Wellenlängen erzeugt wird, welche zur Analyse der entsprechenden Inhaltsstoffe in Blut oder Gewebe oder in einem anderen Messmedium geeignet sind. Typischerweise wird eine Lichtquelle verwendet, die für Patienten-überwachungen (z. B. Messung der Sauerstoffsättigung) wenigstens Licht im Frequenzbereich von 500 nm bis 850 nm und für Blutzuckerbestimmungen wenigstens Licht im Frequenzbereich von 800 nm bis 1200 nm erzeugt. Die Lichtquelle ist insbesondere eine weisse LED, welche für Blutzuckermessungen auch im NIR-Bereich ausreichend Licht erzeugt. Die Lichtquelle dient zum Beaufschlagen wenigstens eines Messbereichs mit dem breitbandigen Licht. Typischerweise ist der Messbereich eine Stelle der Oberfläche eines Lebewesens, insbesondere eines Menschen, beispielsweise an der Fingerbeere oder am Ohrläppchen.

Die Vorrichtung weist ausserdem Mittel zum Auffächern oder Dispergieren des von der Messstelle zurückgeworfenen Analyselichtes nach seinen Wellenlängen auf. Beim Analyselicht kann es sich einerseits um direkt vom Messbereich reflektiertes Licht oder andererseits um an einer andern Stelle nach Transmission durch Gewebe wieder abgegebenes Analyselicht handeln. Die Vorrichtung weist ausserdem einen Sensor-Array zum Aufnehmen des aufgefächerten Lichts auf. Der Sensor-Array ist eine zweidimensionale CMOS-Anordnung. Je nach Anwendung und geeigneten Frequenzbereichen sind auch andere zweidimensionale Sensorarrays, z. B. InGaAs, verwendbar. Die CMOS-Bildsensoren sind hochauflösend und enthalten typisch eine Million oder mehr Bildpixel (hier eingesetzter Sensor 1,6 MPix oder auch 5 MPix).

Ein erster Vorteil bei der Verwendung von Sensorarrays und typischerweise von CMOS-Sensorarrays ist deren einfache Verfügbarkeit. Zweidimensionale Sensorarrays erlauben aber insbesondere auch eine schnellere Messgeschwindigkeit und bessere Signal-/Rauschverhältnisse. Aufgrund der wellenlängenabhängigen Auffächerung des Messlichts wird das Messlicht auf einer Zeile des Sensors abgebildet. Das Messlicht hat aber eine gewisse Breite, so dass das aufgefächerte Messlicht (d. h. das Spektrum) gleichzeitig von mehreren parallel nebeneinander liegenden Zeilen des Sensorarrays erfasst werden kann. Indem parallel eine Mehrzahl von Zeilen des Sensorarrays ausgelesen werden, können die Resultate der einzelnen Zeilen aufaddiert, d. h. die einzelnen Spektren zusammengezählt werden. Typischerweise kann ein Spektrum erzeugt werden, indem die Signale von bis zu 1000 nebeneinander liegenden Zeilen des Arrays aufintegriert werden. Die Vorrichtung weist zu diesem Zweck ausserdem Mittel zum gleichzeitigen Erfassen der Signale einer Mehrzahl von nebeneinander liegenden Zeilen eines zweidimensionalen CMOS-Sensor Arrays auf. Ausserdem ist die Vorrichtung so ausgebildet, dass die Spektren dieser nebeneinander liegenden Zeilen aufaddiert werden.

Erfindungsgemäss werden also zweidimensionale Sensoren nicht verwendet, um eine ortsaufgelöste Messung durchzuführen. Vielmehr werden die nebeneinander liegenden Zeilen verwendet, um in kurzer Zeit mehr Spektren und daher bessere Signale zu erzeugen. Unter paralleler Messung wird hier eine quasi gleichzeitige Messung verstanden. Selbstverständlich ist klar, dass die einzelnen Pixel und Zeilen des Sensors sequenziell ausgelesen werden. Die Abtastfrequenz ist aber so hoch, dass von einer quasi gleichzeitigen Messung der parallelen Zeilen gesprochen werden kann. Damit wird das Auslesen von Teilbildern und damit höhere Geschwindigkeiten erreicht. Bessere S/N-Verhältnisse sind damit im Spektrum erzeugbar.

Je nach Art der Anwendung sind die Anforderungen an Signal-/Rauschverhältnisse unterschiedlich. Bei Monitoring-Anwendungen (wie beispielsweise der Messung der Blutsättigung) interessiert in vielen Fällen nur das arterielle Blut. Der Gewebeanteil interessiert nicht. Aus diesem Grund sollte bei solchen Monitoring-Anwendungen pulsaufgelöst gemessen werden. Zur pulsaufgelösten Messung sollte das Signal-/Rauschverhältnis ausserdem möglichst so sein, dass aus der Differenz der Messung zwischen der Systole und der Diastole ein ausreichend klares Signal erhältlich ist. Eine pulsaufgelöste Messung ist bei der Messung von Blutinhaltsstoffen wie beispielsweise Blutzucker, Fett oder Alkohol weniger wichtig. Beispielsweise bei Blutzucker ist nach relativ kurzer Zeit ein Ausgleich zwischen dem Anteil im arteriellen Blut und dem Gewebeanteil erreicht. Eine pulsaufgelöste Messung ist in diesem Zusammenhang nicht zwingend, kann aber durchaus zur Überprüfung der Messresultate vorteilhaft sein.

CMOS-Bildsensoren werden heute überwiegend in Mobiltelefonen, Überwachungskameras und Digitalkameras eingesetzt. Gerade aus den zwei erstgenannten Anwendungsbereichen sind hochwertige, miniaturisierte Megapixelobjektive verfügbar.

Solche Sensoren sind sehr klein mit typischen Bildkantenseiten von 3 mm. Weiterhin sind sie für den Auslesebereich parametrierbar. So werden bei reduzierter Bildfläche sehr hohe Bildraten von zum Beispiel über 100 Hz ermöglicht, die eine zeitaufgelöste Auswertung des pulsatilen Signals ermöglichen.

Bei CMOS-Sensoren ist in dem Sensor direkt Elektronik integriert. Die optischen Arrays weisen Schaltungen wie z. B. Ausleseschaltungen, einstellbare Verstärker und A/D-Wandler auf. Dies erlaubt es, Daten schnell und mit dünnen Kabeln zu übertragen. Die ganze Anordnung enthaltend Spektrometer, Beleuchtung, Elektronik und Bildaufnahme ist sehr klein (erfindungsgemäss kleiner 20 mm x 30 mm x 100 mm, typischerweise etwas 10 mm x 15 mm x 50 mm) ausgebildet werden. Eine solche Vorrichtung kann daher nur mit einem dünnen elektrischen Kabel versehen und direkt am Patienten befestigt werden. Auf Glasfasern etc. kann verzichtet werden. Aufgrund der Bauformen der CMOS Anordnungen von wenigen Millimetern Größe haben diese gut Platz in einem Miniatursystem, zum Beispiel am Finger oder am Ohrläppchen oder an Stellen, in denen der verfügbare Platz beschränkt ist.

Durch die mittlerweile sehr gute Bildqualität und den niedrigen Lichtbedarf können auch kleine, miniaturisierte Beleuchtungseinheiten eingesetzt werden.

Da bei im Stand der Technik teilweise verwendeten CCD-Arrays immer der volle Detektor ausgelesen werden muss, können keine ausreichend hohen Bildraten erzielt werden, da CCDs Raten von wenigen Hz aufweisen. CMOS-Sensoren können auf eine "region of interest" (ROI) beschränkt und dadurch schneller gemacht werden, da nur diejenigen Daten ausgelesen werden müssen, die gebraucht werden. CMOS-Sensoren haben zwar bei einem Vollbild ebenfalls eine verhältnismässig langsame Bildrate. Bei Beschränkung auf eine ROI können Bildraten bis zu typisch 200 Hz erzielt werden.

Dies erlaubt es auch, die Spektren so schnell aufzunehmen, dass pulsaufgelöst gearbeitet werden kann. Typischerweise beträgt der maximale Puls ca. 3 Hz. Bei einer vierfachen Abtastung wären also ca. 12 Hz erforderlich.

Eine in vivo Messung von Blutparametern kann anhand des pulsatilen Anteils des Signals durchgeführt werden. Durch die Unterscheidung von pulsatilen und statischen Anteilen ist es möglich, die Einflüsse des Blutes von denen des Gewebes zu trennen. Dieser Einfluss und eine mögliche Auswertung hierzu sind auch in DE 195 18 511 beschrieben.

Aufgrund der dikrotischen Inzisur ergibt sich eine Verdoppelung der Grundfrequenz. Da die Fourieranalyse des Frequenzinhaltes der Blutdruckkurve Komponenten bis zur achten Harmonischen beinhalten kann, ist eine Abtastung mit 50 Hz technisch sinnvoll. Eine rasche Abtastung reduziert ausserdem Bewegungsartefakte, welche hochfrequente Signalanteile erzeugen. Bei Verletzung des Abtasttheorems spiegeln sich solche Störungen direkt im Nutzbereich des Signals.

Wenn bei 50 Hz in jeder Bildaufnahme ca. 1000 Spektren auf nebeneinander liegenden Zeilen des Sensors aufgenommen werden, welche durch Aufaddition und -integration eine ausreichende Datentiefe und S/N erzeugen, kann nicht nur der Gewebeanteil, sondern auch noch der pulsatile Anteil (arterielles Blut mit ca. 1% des Signals als Spektrum) spektral nach den Blutkomponenten ausgewertet werden.

Der Sensor-Array ist so angeordnet, dass Licht von unterschiedlicher Wellenlänge an unterschiedlichen Stellen auf den Array auftrifft. Ausserdem wird das aufgefächerte Licht parallel auf mehrere nebeneinander liegende Zeilen des Sensors geleitet.

Das Grundprinzip der erfindungsgemässen Vorrichtung und deren Vorteile sind für die verschiedenen Anwendungen gleich. Je nach den zu messenden Inhaltsstoffen ist der Frequenzbereich anzupassen. Entsprechend sind Lichtquellen, Sensoren, Beugungsgitter und die verwendeten Optiken der Messsituation anzupassen. Die Erfindung wird im Detail anhand der Bestimmung von Blutwerten bei der Patientenüberwachung und anhand der Blutzuckermessung erläutert.

Erfindungsgemäss weist die Vorrichtung ein Gehäuse auf und ist als kompakte Baueinheit ausgebildet. Die kompakte Baueinheit enthält wenigstens die Lichtquelle, die Mittel zum Auffächern des Analyselichtes und den Sensor-Array. Dank dieser Anordnung können das Beleuchtungs- und das Spektroskopiesystem direkt im Sensor an einer Messstelle integriert werden. Die Beleuchtung und das Miniaturspektrometer können direkt am Messbereich angebracht werden. Damit kann auf die relativ starren und großen optischen Fasern verzichtet werden. Es steht ein Vielfaches mehr an Licht zur Verfügung.

Wenn Licht durch eine Glasfaser auf die Messstelle übertragen wird, geht bereits ein grosser Teil der Leistung verloren. Wenn die Glasfaser ins Gewebe eingekoppelt und mit einer anderen Glasfaser das zurückgegebene Licht wieder aufgenommen wird, geht wiederum ein Großteil des Lichtes verloren. Wenn ausserdem nur ein Spalt für ein Spektrometer ausgekoppelt und spektral aufgefächert wird, bleibt zur Detektion nur eine geringe Lichtmenge übrig. Das führt dazu, dass bei bekannten Anordnungen die größten technisch verfügbaren Lampen eingesetzt werden und gleichzeitig mit langen Belichtungszeiten gearbeitet wird.

Im Gegensatz dazu werden bevorzugt eine sehr kleine Lichtquelle (z. B. eine LED) und ein Spektrometer in einem Gehäuse direkt ans Gewebe gebracht. Dies erhöht die Lichtausbeute, sodass die Belichtungszeiten sehr kurz sind.

Die Lichtquelle, die Mittel zum Auffächern des Messlichtes und der Sensor-Array erlauben eine spektroskopische Analyse des Blutes und des Gewebes im Bereich des Messbereichs.

In der Spektroskopie gibt es unterschiedliche Methoden. Eine neue Methode ist der Bereich der bildgebenden Spektroskopie (Spectral Imaging). Dabei wird das Licht über effiziente Gitter/Optik-Anordnungen auf zweidimensionale Sensorarrays spektral zerlegt. So erhält man auf dem Sensor in einer Richtung eine örtliche Information, in der anderen Richtung die spektrale Information. Jeder einzelne Bildpunkt ist dabei ein Pixel, welches eine Intensitätsinformation in üblicherweise 8, 12, 14 oder 16 Bit Datentiefe enthält. Bei dieser Technologie haben sich CMOS-Bildsensoren durchgesetzt, welche gerade für die hier beschriebene Erfindung vorteilhafte Eigenschaften enthalten. Für den langwelligeren Spektralbereich sind hier InGaAs-Sensoren geeignet, welche aber auch einzelne Photoelemente haben, die auf einem CMOS-Unterbau die notwendige Schaltungstechnik integriert haben.

Erfindungsgemäss werden bevorzugt solche Beugungsgitter als Mittel zum Auffächern des Messlichtes und solche CMOS-Sensor Sensor-Arrays zur Aufnahme des aufgefächerten Lichtes verwendet.

Die wellenlängendispersive Einrichtung umfasst also bevorzugt ein dispersives optisches Element, im Allgemeinen ein optisches Gitter, insbesondere ein holografisches Gitter, das in einer vorteilhaften Ausführung ein geblaztes Gitter ist, um eine hohe Lichtausbeute in der von der Kamera bzw. dem Bildsensor erfassten Beugungsordnung und dem geeigneten Wellenlängenbereich von z. B. 500 nm bis 850 nm für Messungen von SpO2-Konzentrationen oder von 800 nm bis 1200 nm für Blutzuckermessungen zu ermöglichen.

Für Blutzuckermessungen wird der Spektralbereich mit ca. 800 nm bis 1200 nm definiert. In diesem Spektralbereich wurden die stärksten Signalvariationen im Bereich 960 nm +/-50 nm und 1150 nm +/-50 nm detektiert. In den Spektren zeigt sich die Korrelation mit sich ändernden Wassersignalen. Mit der InGaAs-Sensortechnologie lassen sich gleichzeitig beide Bereiche auswerten. Allerdings sind die derzeit kommerziell verfügbaren Sensoren in diesem Spektralbereich deutlich schlechter als die CMOS-Sensoren, die jedoch nur bis 1100 nm Licht empfangen. So haben die InGaAs-Sensoren deutlich kleinere Pixelanzahlen (typisch 100k Pixel bis 1000k Pixel) und damit eine schlechtere S/N-Ratio.

Die maximale Beugungseffizienz kann so gewählt werden, dass sie in den Wellenlängenbereich fällt, in dem der eingesetzte Sensor die niedrigste Sensitivität besitzt. Das Blaze-Gitter kann z. B. ein Transmissionsgitter mit asymmetrisch sägezahnförmigem Gitterprofil sein, wobei die Sägezahnflanken jeweils als einzelne Spiegel derartig ausgebildet sind, dass sie das Licht in Richtung der gewünschten Beugungsordnung transmittieren. Weiterhin können auch holografische Gitter eingesetzt werden. So können z. B. VPH-Gitter (Volume phase holographic gratings) als spezifische Blaze- bzw. holografische Gitter eingesetzt werden. Diese VPH-Gitter sind Transmissionsgitter, bei denen ein transparentes, photoempfindliches Material zwischen zwei Glas- oder Kunststoffscheiben eingeschlossen ist, in welchem ein gewünschtes Muster eines variierenden Brechungsindex erzeugt wurde, z. B. durch holografische Belichtung und dadurch erfolgende Strukturänderung des Materials. Erfindungsgemäß können durch Einsatz derartiger Blazegitter hohe Effizienzen von über 80% der Beugungsintensität in einem kleinen, vorgegebenen Wellenlängenbereich erzielt werden.

So kann mit einem Beugungsgitter und einem Eintrittsspalt ein sehr kleines Spektroskopiesystem erstellt werden, welches zum einen den gesamten Spektralbereich abdeckt und zum andern eine Zeitauflösung besitzt, die für die pulsabhängige Aufnahme wichtig ist. Weiterhin können durch die zweidimensionale Bilderfassung viele Spektren gleichzeitig aufgenommen und ausgewertet werden, was zu einer erheblichen Verbesserung des Signal-/Rauschverhältnisses führt.

Diese Technologiekombination ermöglicht es, kleine, hochauflösende und sehr schnelle Sensoreinheiten zu konstruieren, die direkt an die für die Pulsoximetrie üblichen Körperstellen angebracht werden können.

So sind Sensoren möglich, die an bevorzugten Messstellen wie Fingerbeere, Handballen oder Ohrläppchen oder auf Hautflächen befestigt werden können. Besonders bevorzugt ist daher das Gehäuse zum Anbringen an einer Körperstelle eines menschlichen Patienten, insbesondere an Fingern oder Ohrläppchen, ausgebildet.

Die Messqualität im Körper hängt maßgeblich von der ausgewählten Messstelle ab. Die Stelle muss insbesondere zur Blutzuckermessung gut durchblutet sein, sollte wenig Fettgewebe enthalten und für Messungen einfach erreichbar sein. Daher bieten sich vor allem für die Blutzuckermessung die folgenden Messstellen in der genannten Reihenfolge an: Transmission durch Finger, Handballen oder Ohrläppchen. Beim Finger ist darauf zu achten, dass man möglichst ohne Knochen und Fingernagel misst. Daher bietet es sich an, das Licht seitlich in den Finger einzukoppeln und auf einer Linie zentral auf der Fingerbeere abzugreifen.

Diese vorgeschlagene Kombination von Technologien ermöglicht folgende, sehr wichtigen Sensoreigenschaften: Die Sensoren besitzen eine Pixelauflösung, die es ermöglicht, ein gesamtes Spektrum in der notwendigen spektralen Auflösung unter ca. 5 nm aufzunehmen.

Es besteht die Option, Teilbereiche des Sensors auszulesen und somit hohe Ausleseraten (typisch größer 100 Hz) aufzunehmen und auszulesen und somit pulsabhängig die spektralen Gewebe- und Bluteigenschaften auszuwerten.

Erfindungsgemäss weist die Vorrichtung ausserdem eine Spaltblende auf. Die Spaltblende ist zwischen einem Eintrittsbereich für das Analyselicht und dem Mittel zum Auffächern des Analyselichtes angeordnet. Die Spaltblende erlaubt es, einen Messbereich genau zu definieren. Insbesondere ist die Spaltbreite bezogen auf die Mittel zum Auffächern so angeordnet, dass ein längliches Bild in einer zur Ausdehnung des Bildes verschiedenen Richtung, vorzugsweise senkrecht dazu, aufgefächert wird. Auf diese Weise lässt sich auf einem zweidimensionalen Sensor-Array in einer Richtung eine nach Wellenlängen aufgelöste Darstellung und in der andern Richtung eine ortsaufgelöste Darstellung aus dem Messbereich erzielen. Besonders bevorzugt ist die Vorrichtung ausserdem direkt mit einem A/D-Wandler versehen. Typischerweise verfügen heutige CMOS-Bildsensoren bereits solche A/D-Wandler. Die ortsaufgelöste Darstellung wird allerdings erfindungsgemäss nicht zur ortsaufgelösten Analyse verwendet. Vielmehr dient die parallele Messung von mehreren Spektren durch nebeneinander liegende Zeilen zur Verbesserung des Signals.

Vorliegend werden als Blende sämtliche optischen Mittel verstanden, die einen lang gestreckten, streifenförmigen Bereich des über die erste Abbildungsoptik (Objektiv) abgebildeten Bereichs ausblenden. Hierbei ist der streifenförmige Bereich nicht notwendigerweise durchgängig, sondern kann z. B. auch aus einer Folge von einzelnen Bildelementen zusammengesetzt sein.

Die Vorrichtung weist ausserdem bevorzugt einen Verstärker für die Signale auf, der von aussen parametriert werden kann. CMOS-Bildsensoren weisen häufig bereits solche von aussen parametrierbare, integrierte Verstärker auf.

Dank der Digitalwandlung der Signale auf der Schaltung können die digitalen Signale über größere Entfernungen einfach verlustfrei elektrisch zu einer Auswerteeinheit übertragen werden.

Die Lichtquelle ist bevorzugt eine LED. LEDs sind sehr schnell schaltbare Lichtquellen (typisch 10-1000 μs). Sie arbeiten ohne thermische Probleme mit hohen, aber für das Gewebe unkritischen Lichtleistungen.

Zum Einsatz bei der Patientenüberwachung (z. B. Sauerstoffsättigung im Blut) wird bevorzugt Licht im sichtbaren (VIS) und nahinfraroten (NIR) Spektralbereich, insbesondere im sehr nahen Infrarotbereich, z. B. im VNIR-Bereich von 500 nm bis 850 nm, eingesetzt. Dieses Licht wird vorzugsweise durch eine LED oder eine Kombination von LEDs erzeugt. Hierfür eignen sich zum Beispiel übliche Weißlicht-LEDs, die eine breitbandige Lichtemission durch einen zusätzlichen überlagerten Fluoreszenzfarbstoff haben. Als Farbstoffe können hier anorganische Fluoreszenzfarbstoffe zum Einsatz kommen, die zum Beispiel Ytterbium oder andere seltene Erden in YAG oder ähnliche Wirtsgitter haben.

Durch Kombination unterschiedlicher Farbstoffe lässt sich Licht im gesamten, je nach Anwendung benötigten Spektralbereich erzeugen, so zum Beispiel für Blutzuckermessungen auch im Bereich von 800 nm bis 1200 nm. Es ist aber auch möglich, Licht von unterschiedlichen LEDs zu kombinieren. Hierbei ist aber zu beachten, dass die Emitter temperaturstabilisiert sein müssen und die Strahlung örtlich gut homogenisiert sein muss.

Die Vorrichtung weist weiterhin bevorzugt einen Anschluss für elektrische Kabel auf. Insbesondere weist die Vorrichtung ausserdem bevorzugt keine Anschlüsse für zusätzliche optische Leitungen zum Zuführen oder Ableiten von Licht auf. Ein dünnes Kabel mit einigen elektrischen Adern ist für den Betrieb der erfindungsgemässen Vorrichtung ausreichend, insbesondere weil für die erfindungsgemässe Lichtquelle und für die erfindungsgemässen Sensoren keine hohen Ströme und Spannungen erforderlich sind und insbesondere wenn Kabel nicht für analoge Signale geschirmt werden müssen.

Dank der Aufnahme der gesamten Spektren ist die Ermittlung und Überwachung einer Vielzahl von verschiedenen physiologischen Blutwerten möglich. Insbesondere ist die Auswertung der folgenden Parameter möglich:

Pulsfrequenz
Pulsform und Struktur
Sauerstoffsättigung Hb (SHbO2)
Gesamt-Hb (ctHb)
HbCO-Konzentration
Konzentration MetHb
Konzentration des desoxigenierten Hb
PI (Perfusionsindex)
PVI (Pleth Variability Index)
Gewebesauerstoffsättigung StO2
Konzentration des Blutzuckers
Laktose

Physiologische Blutwerte sind im Rahmen der vorliegenden Beschreibung alle Werte, welche bei einem Patienten zu diagnostischen Zwecken oder zur Überwachung ermittelt werden, insbesondere die vorstehend aufgeführten Werte.

Ausserdem sind auch verschiedene Anwendungen im nichtmedizinischen Bereich möglich, wie beispielsweise die Überwachung von Verbrennungsprozessen (durch Messung von Prozessgasen), bei der Produktion beispielsweise von Lebensmitteln oder pharmazeutischen Produkten bei der Zudosierung von Inhaltsstoffen.

Besonders bevorzugt ist die Vorrichtung zur Anwendung sowohl zur Transmissions- als auch zur Reflexionsmessung ausgebildet. Damit können Blutkomponenten im sichtbaren Spektralbereich in Reflexion und im VNIR in Transmission gemessen werden, um die starke Absorption zwischen 500 nm und 850 nm zu kompensieren.

Wenn ausreichend Licht eingestrahlt werden kann (oder auch je nach gemessener Wellenlänge) ist es auch denkbar, nur in Transmission zu messen. Insbesondere bei Glukosemessungen oberhalb von 800 nm ist das Problem der starken Absorption im Bereich 500 nm bis 850 nm nicht mehr so gross. Bei ausreichend grosser eingestrahlter Lichtmenge sind aber rein transmissive Messungen auch beim Monitoring, beispielsweise bei der Messung der Sauerstoffsättigung, denkbar.

Für die kombinierte Aufnahme in Reflexion und Transmission gibt es unterschiedliche Möglichkeiten der Umsetzung.

In einer ersten Ausführungsform für die Überwachung von Patienten wird eine zeitliche Aneinanderreihung der Reflexions- und Transmissionsaufnahmen durchgeführt. Dabei wird abwechselnd Licht auf zwei Bereiche der Haut eingestrahlt. Zuerst wird Licht im Bereich einer linienförmigen Aufnahmestelle eingestrahlt und das Reflexionsbild ausgelesen. In einem nächsten Schritt wird Licht an einer oder mehreren Stellen außerhalb der Aufnahmelinie eingestrahlt und das bis zur Aufnahmelinie transmittierte Licht aufgenommen und ausgelesen. Beide Informationen werden in der Auswerteeinheit miteinander verknüpft. Insbesondere ist dazu die Vorrichtung mit einer Rechneranordnung versehen, welche so ausgestaltet ist, dass alternierend eine Transmissions- und eine Reflexionsmessung durchführbar ist. Ausserdem weist die Vorrichtung zu diesem Zweck eine Lichtquelle auf, welche die Einstrahlung von Licht an zwei verschiedenen Messstellen ermöglicht. Dies kann durch die Verwendung von mehreren Lichtquellen oder durch die Verwendung von geeigneten Umlenkmitteln erfolgen.

In einer zweiten Ausführungsform für die Überwachung von Patienten wird eine örtliche Trennung der Reflexions- und Transmissionsbereiche durchgeführt. Dazu weist die Vorrichtung und insbesondere deren Gehäuse Mittel zum Trennen des Analyselichts aus einem Reflexionsbereich und einem Transmissionsbereich auf. Das einfallende Licht wird auf einen Teil der Haut gestrahlt, der im Sichtbereich des Sensors liegt. Der zweite Teil des Sichtbereichs des Sensors wird durch eine mechanische Blende von dem eingestrahlten Licht getrennt. Somit kann in diesen Bereich nur Licht dringen, welches durch das menschliche Gewebe hindurchgetreten ist.

Das reflektierte und von der Haut nach der Transmission austretende Licht kann über ein Objektiv abgebildet werden und es kann mit einer länglichen Blende (Spalt) zunächst ein im Wesentlichen längliches bzw. eindimensionales Bild extrahiert werden, das nachfolgend in einer hierzu verschiedenen, vorzugsweise hierzu senkrechten Richtung wellenlängendispersiv aufgefächert, insbesondere gebeugt werden kann. Dadurch kann auf einfache Weise mit relativ einfachen Mitteln ein zweidimensionales Bild erzeugt werden, das wellenlängenaufgelöste Informationen über den linienförmig erfassten Haut- und Gewebebereich liefert. Indem die Strahlung von einem Bildsensor bzw. Bildwandler erfasst wird, wird eine nachfolgende Analyse ermöglicht, sodass die in der Haut und im Gewebe enthaltenen Substanzen quantitativ und pulsabhängig ermittelt werden können und somit in kurzer Zeit Aussagen über die Zusammensetzung, insbesondere die chemische Zusammensetzung des Blutes, mittels einer in vivo Messung ermöglicht werden.

Somit kann erfindungsgemäß die Funktionalität einer zeitaufgelösten und pulsabhängigen Sensoraufnahme mit einer spektroskopischen Untersuchung und Analyse kombiniert werden. Das Licht kann durch den Aufbau zum einen im Reflexions-, zum anderen im Transmissionsbereich erfasst werden.

Die Bestimmung des Blutzuckergehaltes erfolgt auf vergleichbare Weise, wobei eine reflexive Messung nicht zwingend ist.

Erfindungsgemäß kann der Spalt im Wesentlichen der Linienrichtung der Aufnahmestelle auf der Haut entsprechen. Die Beugungsrichtung bzw. wellenlängendispersive Richtung kann dann senkrecht zu dieser Spaltrichtung verlaufen, so dass die Zeilen und Spalten eines zweidimensionalen Pixelarrays des Bildsensors diesen Richtungen entsprechen können. Somit ergibt sich ein Bild mit eindimensionaler Ortskomponente entsprechend z. B. der Aufnahmelinie auf der Haut und hierzu orthogonaler Beugungsrichtung zur Ermittlung eines Beugungsbilds und der relevanten Spektren.

Das Linsensystem ist vorteilhafterweise mit miniaturisierten Objektiven ausgebildet. Hierzu können entweder Megapixelobjektive aus dem Bereich der Überwachungskameratechnik eingesetzt werden oder Miniaturobjektive (z. B. polymerbasierte Objektive), die in Kameras von Mobiltelefonen einen breiten Einsatz haben. Alternativ können aber auch andere Linsensystem oder auch Achromaten für die Abbildung eingesetzt werden.

Diese Objektive sind gut mit den eingesetzten, sehr kleinen Sensoren zu kombinieren. Verzerrungen, welche diese sehr kleinen Objektive häufig aufweisen, können durch ihre statische Natur softwaretechnisch ausgeglichen werden.

Die erfindungsgemäße Vorrichtung kann z. B. drei Abbildungsoptiken bzw. Objektive aufweisen. Hiervon erzeugt die erste Abbildungsoptik eine zweidimensionale Abbildung des ausgeleuchteten Bereichs auf die in vorzugsweise der Bildebene dieser ersten Abbildungsoptik angeordnete, längliche bzw. spaltenförmige Blende. Die zweite Abbildungsoptik bildet die spaltförmige Blende dann z. B. ins Unendliche ab, so dass sie der Kollimation des durch den Spalt hindurchtretenden Lichtstreifens dient. Hinter dieser zweiten Abbildungsoptik ist die wellenlängendispersive Einrichtung mit dem vorzugsweise optischen Gitter angeordnet, das die dispersive Aufspaltung des Lichts in der zweiten Richtung ermöglicht.

Das dritte Objektiv erzeugt wiederum eine Rücktransformation des nun bereits wellenlängendispersiv aufgespaltenen Blendenabbildes. Somit erhält man auf dem Sensor ein wellenlängenaufgefächertes Abbild der auf der Haut aufgenommenen Linie.

Erfindungsgemäß kann somit der Bildsensor in dem auf die jeweilige Anwendung optimierten Wellenlängenbereich positioniert werden und kann dort z. B. lediglich einen relativ kleinen Raumwinkelbereich abdecken.

Durch die erste Abbildungsoptik kann eine Abbildung des zu analysierenden Bereichs auf den Spalt der Blende erreicht werden, so dass die Blende Bereiche außerhalb der Aufnahmelinie effektiv ausblendet. Somit ist es durch den Einsatz der Blende grundsätzlich auch möglich, mit der Anordnung einen etwas größeren Bereich auszuleuchten als den nachfolgenden spektroskopisch untersuchten Bereich, der durch die Blende begrenzt wird.

Die LED-Beleuchtungsquelle wird bevorzugt gepulst gesteuert. Dadurch können Beeinflussungen durch Fremdlicht reduziert werden.

Ausserdem kann ein interner Schwarzwertabgleich erfolgen. CMOS-Sensoren der neueren Generationen haben einen internen Schwarzabgleich. An den Rändern sind Pixel schwarz abgedeckt. Diese werden intern mitausgelesen und intern für eine Scharzwertnormierung eingesetzt. Dies behebt zwar nicht das Problem von Fremdlicht, aber die üblichen Probleme, dass Sensoren bei Temperaturschwankungen oder Schwankungen in der Versorgungselektronik driften. Damit können Bilder mit sehr kurzen Belichtungszeiten und hohen Lichtintensitäten aufgenommen werden. Daher sind Fremdlichteinflüsse generell niedrig. Falls Fremdlichteinflüsse auftreten können, kann jeweils zusätzlich ein Hintergrundbild ohne LED-Beleuchtung und mit stark reduzierter ROI (region of interest; untersuchter Frequenzbereich) aufgenommen werden und das Bild damit korrigiert werden. Vor dem Einsatz des Sensors wird in der Auswerteeinheit ein festes Weißbild der Beleuchtung hinterlegt. In Formel (1) entspricht dies $I_0(\lambda)$.

Nach jeder Aufnahme werden alle ca. 500 bis 1000 nebeneinander liegenden, ortsaufgelösten Spektren zu einem Spektrum mit einer hohen Datentiefe aufaddiert, und es wird nach der vorstehenden Formel 5 der Wert $I(\lambda)$ erzeugt. Weiterhin wird die zweite Ableitung eines des aufaddierten Spektrums erzeugt. Hiermit können direkt die notwendigen Konzentrationen bestimmt werden. Bei der Auswertung der zeitabhängigen Werte können - wie in der Pulsoximetrie üblich - aus dem pulsatilen Anteil nun auch die Werte für den Anteil des arteriellen Blutes bestimmt werden. Wenn man nun die Möglichkeit hat, die spektroskopischen Daten pulsaufgelöst zu ermitteln, ist es zur Blutzuckerbestimmung auch möglich, die Spektren der Systole und der Diastole getrennt aufzuintegrieren und durch einfache Differenzbildung das reine Spektrum vom arteriellem Blut zu erhalten und somit nicht den Blutzuckeranteil im Gewebe zu bestimmen, sondern den Blutzuckeranteil im arteriellen Blut innerhalb des Körpers.

Das aufgenommene Spektrum variiert in Abhängigkeit des Druckes, mit dem der Finger (oder eine andere Messstelle) auf den Sensor gedrückt wird. Wenn die zweite Ableitung des Spektrums analysiert wird, wird diese Druckabhängigkeit vermieden. Ausserdem erlaubt es die zweite Ableitung, die Absorption nur in Bezug auf das arterielle Blut zu messen. Die Effekte aus Lichtstreuung im umliegenden Gewebe werden vermieden. Der Effekt des Anpressdrucks auf das Spektrum ist unter Umständen grösser als die Absorption durch das arterielle Blut. Es ist daher wichtig, dass Messungen vorgenommen werden können, welche nicht durch den Anpressdruck beeinflusst werden. Dies ist bei der Analyse der zweiten Ableitung möglich.

Für die unterschiedlichen Auswertungen können auch unterschiedliche Bereiche aufsummiert oder Teilbereiche unterschiedlich beleuchtet und unterschiedlich analysiert werden.

Für die Pulsinformation ist es vorteilhaft, größere Bereiche der wellenlängenaufgespalteten Information gerade im Bereich 520 nm bis 570 nm zusammenzufassen und auszuwerten. Da der Puls im gesamten Spektralbereich vorhanden ist, ist es alternativ möglich, für die Auswertung alle Pixel aufzusummieren. So können zum Beispiel bei einer Abtastrate von 50 Hz typisch jeweils 500'000 Bildpunkte mit 12 bit Datentiefe pro Bild aufintegriert werden, was eine sehr große Datentiefe ergibt und somit auch sehr schwache Variationen der Intensität durch den Pulsschlag detektierbar macht.

Aus der Pulsinformation kann in dem Verhältnis der Pulsamplitude zu der festen, nicht zeitveränderlichen Absorption durch das Gewebe und dem venösem Blut der Perfusionsindex PI nach der Formel

$$PI = \frac{AC}{DC} * 100\% \qquad (7)$$

erfasst werden. Hierbei ist AC die Amplitude des pulsabhängigen Signals und DC das maximale Absorptionssignal. Dieser PI-Wert ist abhängig von der Wellenlänge, kann aber nach der Veröffentlichung " The wavelength dependence of pulse oximetry" (Damianou, D.; Crowe, J.A.; Pulse Oximetry: A Critical Appraisal, IEE Colloquium; Volume 1996, Issue 124, 29 May 1996 Page(s):7/1 - 7/3) skaliert werden.

Diese Messgröße gibt bei der Veränderung frühzeitig einen Hinweis auf unterschiedliche, klinisch relevante Veränderungen im Patientenzustand.

Eine weitere wichtige Messgröße ist der "Pleth Variability Index" (PVI), der eine Korrelation zwischen Atmung und Puls herstellt. Der PVI wird in Vielfachen des Atemzyklus bestimmt durch die Formel

$$PVI = \frac{PI_{max} - PI_{min}}{PI_{max}} * 100\% \qquad (8).$$

Die Berechnungen der %SpO2-Konzentration und der Gesamthämoglobinwerte kann wie in "The light-tissue interaction of pulse oximetry" (Mannheimer Ph.D.; Anesth. Analg. 2007 Dec; 105(6 Suppl): S10-7. Review) oder "LED Based Sensor System for Non-Invasive Measurement of the Hemoglobin Concentration in Human Blood" (U. Timm, E. Lewis, D. McGrath, J. Kraitl and H. Ewald; 13th International Conference on Biomedical Engineering; Volume 23, Springer Berlin Heidelberg, 2009) beschrieben durchgeführt werden.

Bei der Auswertung können vergleichbar zur konventionellen Oximetrie zwei Spektralbereiche verglichen werden. Zum Beispiel können zum Erzeugen des üblichen Signals bei 660 nm die Spektralkanäle zwischen 640 nm und 680 nm aufintegriert werden. Effektiv können hierbei zum Beispiel typisch 50'000 Pixel zur Erzeugung des spektralen Messpunktes aufintegriert werden.

Die bevorzugte Auswertung ist aber die chemometrische Auswertung des pulsatilen Spektrums.

Die Konzentrationen der unterschiedlichen Hämoglobinderivate werden direkt aus der quantitativen, spektroskopischen Analyse bestimmt.

Absorptionsmaxima oder -minima in der zweiten Ableitung:

| HbO2 | 542 nm | 576 nm |
|---|---|---|
| Hb | 555 nm | 754 nm |
| HbCO | 538 nm | 569 nm |
| MetHb | 640 nm | |
| MbO2 | 545 nm | 580 nm |
| Mb | 558 nm | 758 nm |
| MetMb | 628 nm | |
| | | |
| H2O | 730 nm | 830nm |

In einer Auswerteeinrichtung können die sehr schnell hintereinander erzeugten Spektralinformationen ermittelt werden, wobei z. B. eine in der Spektroskopie übliche multivariate statistische Analyse durchgeführt werden kann, um die spektralen charakteristischen Reflexions- oder Absorptionsanteile aus dem erfassten Spektrum zu bestimmen. Hierbei können unterschiedliche multivariate statische Analyseverfahren eingesetzt werden, z. B. Korrelation, Regression, Variantenanalyse, Diskriminanzanalyse sowie Hauptkomponentenanalyse (PCA).

Die zeitaufgelöste Auswertung kann in einer von der Vorrichtung getrennten Auswerteeinrichtung erfolgen. Wenn in der erfindungsgemässen Vorrichtung die Messwerte digitalisiert und über eine elektrische Verbindung an eine zentrale Auswerteinheit übergeben werden, können die Übertragungskabel dünn gehalten werden. Gleichzeitig ist es nicht erforderlich, den Sensor bzw. die erfindungsgemässe Vorrichtung aufgrund von notwendigen Rechnern, Ein- oder Ausgabegeräten so gross auszubilden, dass ein Anbringen an den Messorten nicht mehr möglich ist. Eine externe Auswerteeinrichtung bietet auch die Möglichkeit temporärer Datenspeicherung oder Datenauswertung mittels komplexeren, mathematischen Verfahren.

Durch die zeitabhängige Auswertung kann zwischen Informationen vom Gewebe und arteriellem Blut unterschieden werden. Diese Blutinformation kann zum einen von der theoretischen Seite mittels bekannter Extinktionskoeffizienten präzise erfasst werden. Zum anderen kann in dem analysierten Spektralbereich auch eine sehr schwache Wasserbande bei ca. 730 nm ausgewertet werden. Da die Konzentration von Wasser im Blut immer sehr präzise im Bereich von 80 bis 85 Volumenprozent liegt, lässt sich bei jedem Messsignal anhand der Auswertung eine zweite, unabhängige Eichung der Messung durchführen. Die Vorrichtung oder insbesondere die Auswerteinrichtung kann daher ausserdem zum Bestimmen von Absolutwerten der Konzentrationen über das Wassersignal ausgebildet sein.

Ein weiterer Vorteil der kombinierten reflexiven und transmissiven, spektral aufgelösten Methode ist, dass das Pulssignal stabiler erfasst werden kann. Die durchschnittliche Differenz des Signals zwischen Pulsmaximum und Pulsminimum ist im sichtbaren Spektralbereich im Verhältnis zum Grundsignal im Bereich 570 nm deutlich höher als im VNIR-Spektralbereich. Dieser Unterschied kann bis zu Faktor 5 größer sein. Weiterhin können bei der Pulsauswertung interessante Spektralbereiche aufintegriert werden, und so kann auch bei Einzelaufnahmen eine sehr hohe Signaltiefe erreicht werden, die mit Einzelsensoren technisch nur sehr schwierig umsetzbar ist.

Bei der Anwendung der Erfindung zur Blutzuckermessung können sehr niedrige Glukosekonzentrationen durch verbessertes S/N durch die 2D-Spektroskopie erzielt werden. Typischerweise werden bis zu 1 Mio. Spektren integriert. Es bestehen keine Probleme mit Haut, da durch den gewählten Spektralbereich genügend Eindringtiefe ins Gewebe erzielt wird. Die Messung erfolgt zeitaufgelöst. Die hochaufgelöste, spektrometrische Erfassung dient ausserdem zur Auswertung und Rückrechnung mittels Absorptionssignalen und der zweiten Ableitung der Absorptionssignale. Die zweite Ableitung minimiert Einflüsse der Gewebestreuung bei der Auswertung.

Es wird der gesamte typische Spektralbereich von 800 nm bis 1200 nm zur Auswertung der Konzentrationen des Wassers und der Glucose aufgenommen. Die Auswertung im Rohspektrum aber auch in der zweiten Ableitung ist möglich.

In der zweiten Ableitung kann zwischen Messwerten aufgrund des in dem Gewebe gestreuten Messlichts und Messwerten aus dem Blut differenziert werden.

Es können chemometrische Methoden wie PCA und PLS2 zur genaueren quantitativen Auswertung eingesetzt werden. Auch hier kann ein CMOS-Bildsensor für die Einstellung ROI, A/D-Wandlung integriert werden.

Der Bildsensor ist zusammen mit der Auswerteeinrichtung und gegebenenfalls einer Steuereinrichtung sowie gegebenenfalls einer Speichereinrichtung für Referenzdaten bereits in einem Halbleiterbauelement monolithisch integriert, sodass eine kompakte und kostengünstige Ausbildung möglich ist und aufwendige zusätzliche Verdrahtungen entfallen oder gering gehalten werden können.

Das von den Beleuchtungseinrichtungen emittierte Licht bzw. die emittierte Strahlung ist vorzugsweise spektral homogen über den zu messenden Wellenlängenbereich verteilt. Hierbei sendet die Beleuchtungseinrichtung vorzugsweise kollimiertes Licht aus. Als Beleuchtungseinrichtung können unterschiedliche, breitbandige LEDs eingesetzt werden. Es kann auch eine Lichtquelle mit LEDs anderer Wellenlängen und einem zusätzlichen überlagerten Fluoreszenzfarbstoff eingesetzt werden, der in dem Spektralbereich von 500 nm bis 850 nm für Patientenüberwachung bzw. im Bereich von 800 nm bis 1200 nm für Blutzuckermessungen eine breitbandige Emission erzeugt.

Die Beleuchtungseinrichtung bzw. Lichtquelle könnte kontinuierlich sein, sollte vorteilhafterweise aber zeitlich gepulst betrieben werden. Ein gepulster Betrieb hat hierbei den Vorteil, dass die Aufnahmeeinrichtung zum einen unabhängiger von wechselnden Fremdlichteinflüssen ist, und zum anderen nur sehr kurze Zeitmomente aufgenommen werden.

Erfindungsgemäß können die Spektren bei der Analyse insbesondere in Form ihrer weißnormierten Rohspektren und zusätzlich ihrer zweiten Ableitungen ausgewertet werden, wodurch das Verfahren unabhängiger von geräteabhängigen Einflüssen wie Beleuchtungsschwankungen oder auch breitbandigen, parasitären Absorptionen durchgeführt werden kann, die durch unterschiedliche Melaninkonzentrationen in der Haut oder in Gewebestrukturen überlagert sein können.

Ein weiterer Aspekt der Erfindung betrifft die Verwendung der vorstehend beschriebenen Vorrichtung in einem Verfahren zum Erkennen und Überwachen von physiologischen Blutwerten eines Lebewesens in nichtinvasiver in vivo Messung.

Dabei wird in einem ersten Schritt der Sensor mit dem Gehäuse an einem Messbereich angebracht. Der Messbereich ist typischerweise ein Finger oder ein Ohrläppchen.

Es können damit auch Körperstellen im Körperzentrum gemessen werden, da die Pulserkennung in den Extremitäten in bestimmten Zuständen (wenn der Körper die Funktion auf den Kernbereich beschränkt) nur an diesen Stellen möglich ist.

Der Messbereich wird anschliessend mit Licht aus der breitbandigen Lichtquelle beaufschlagt, welche in dem Gehäuse angeordnet ist.

Von der Messstelle zurückgeworfenes Analyselicht wird anschliessend in Reflexion und/oder in Transmission erfasst. Das erfasste Analyselicht wird dann wellenlängenabhängig aufgefächert, und die einzelnen, wellenlängenabhängigen Anteile des erfassten Lichtes werden auf den im Gehäuse angeordneten, zweidimensionalen CMOS Sensor-Array abgebildet. Weiter wird das Licht auf mehreren parallelen Zeilen des Arrays abgebildet. Die durch die parallelen Zeilen erzeugten Spektren werden dann aufaddiert. Das auf diese Weise erzeugte Spektrum wird anschliessend zwecks Bestimmung von physiologischen Blutwerten ausgewertet. Besonders bevorzugt wird das zurückgeworfene Analyselicht an einem Beugungsgitter aufgefächert. Dadurch lassen sich besonders kompakte Vorrichtungen bereitstellen.

Besonders bevorzugt ist es, dass das zurückgeworfene Analyselicht sowohl in Reflexion als auch in Transmission erfasst wird. Dies kann zeitlich nacheinander durch abwechselnde Beleuchtung von unterschiedlichen Messstellen oder parallel durch Erfassung des zurückgeworfenen Lichtes aus verschiedenen Messbereichen erfolgen.

Erfindungsgemäss wird das erfasste Licht zeitaufgelöst ausgewertet. Damit kann eine Vielzahl von weiteren Werten ermittelt und berücksichtigt werden. Besonders bevorzugt ist es, zur Auswertung die zweite Ableitung des erfassten Spektrums oder der erfassten Spektren zu bestimmen. Weiter bevorzugt wird gleichzeitig bei der Auswertung der Wasseranteil im Blut ermittelt und es werden Absolutwerte der Konzentrationen aufgrund des ermittelten Wasseranteils bestimmt.

Die Erfindung wird im Folgenden anhand der beiliegenden Zeichnungen an einigen Ausführungsformen erläutert. Dabei zeigen:

Fig. 1a und 1b      eine schematische Darstellung einer erfindungsgemäßen Vorrichtung in Seitenansicht (Figur 1a) und in Draufsicht (Figur 1b);

Fig. 2      eine schematische Darstellung einer Sensoreinheit;

Fig. 3a und 3b      eine erfindungsgemässe Anordnung zur kombinierten Aufnahme der reflexiven und transmissiven Eigenschaften in zeitlicher Diskriminierung (Figur 3a) und örtlicher Diskriminierung (Figur 3b);

Fig. 4      ein Blockdiagramm der erfindungsgemäßen Vorrichtung;

Fig.5      Aufteilung der Absorptionssignale nach Ursprung;

Fig. 6a und 6b      die spektrale Verteilung einer Weißlicht-LED-Beleuchtung (Figur 6a) sowie das Absorptionsspektrum von Wasser im Spektralbereich 600 nm bis 850 nm und in der zweiten Ableitung (Figur 6b); und

| Fig. 7 | Spektren unterschiedlicher Blutbestandteile als Absorptionsdarstellung und in ihrer ersten und zweiten Ableitung. |
| --- | --- |
| Fig. 8 | Darstellung von verschiedenen Spektren mit verschiedenen Integrationen und für zwei Typen von Sensoren (links 38 dB/rechts 64 dB) |
| Fig. 9 | schematische Darstellung eines nichterfindungsgemässen Sensors |
| Fig. 10 | Darstellung von typischen Spektren |
| Fig. 11 | Darstellung der zweiten Ableitung von typischen Spektren. |

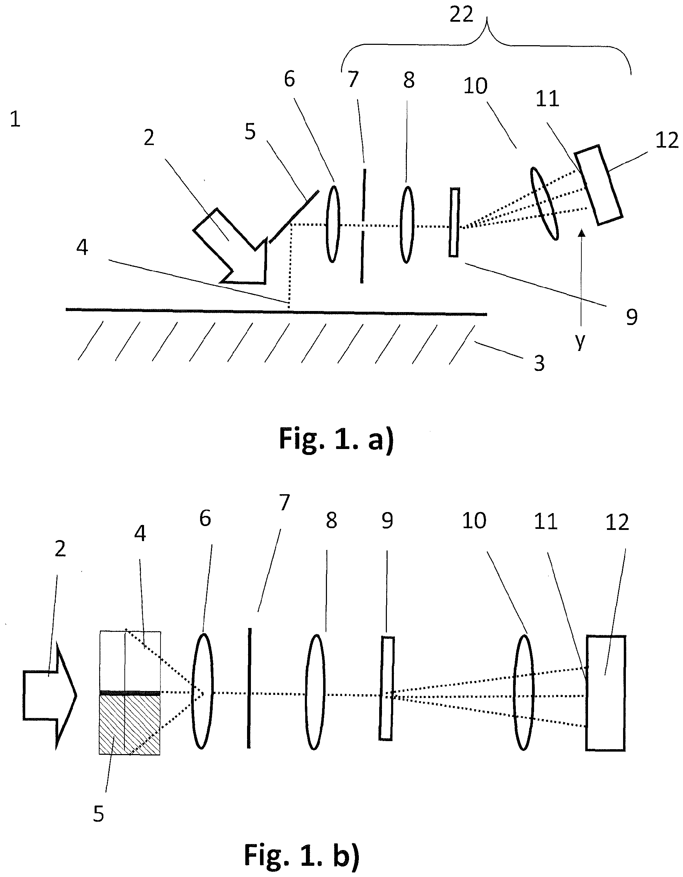

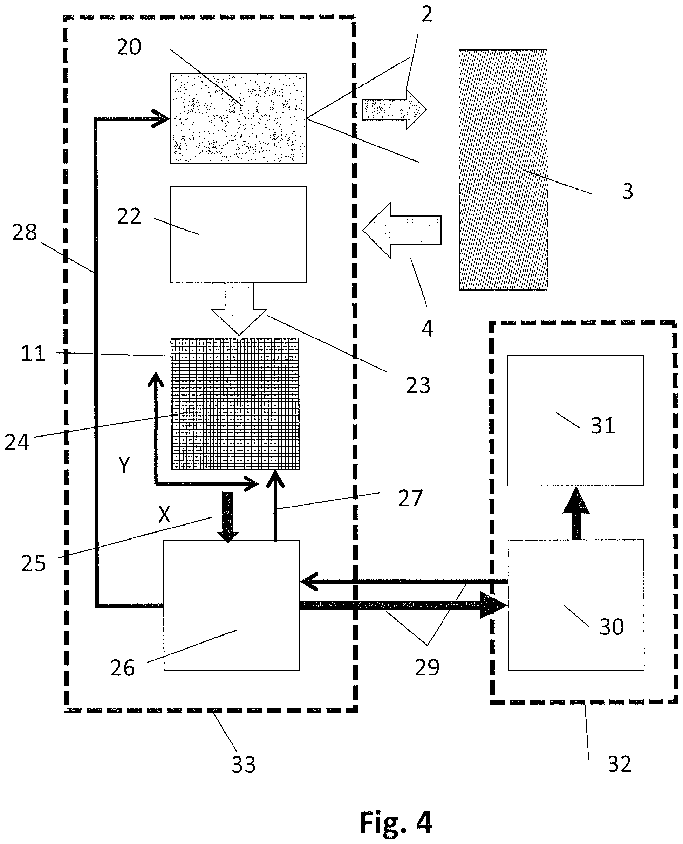

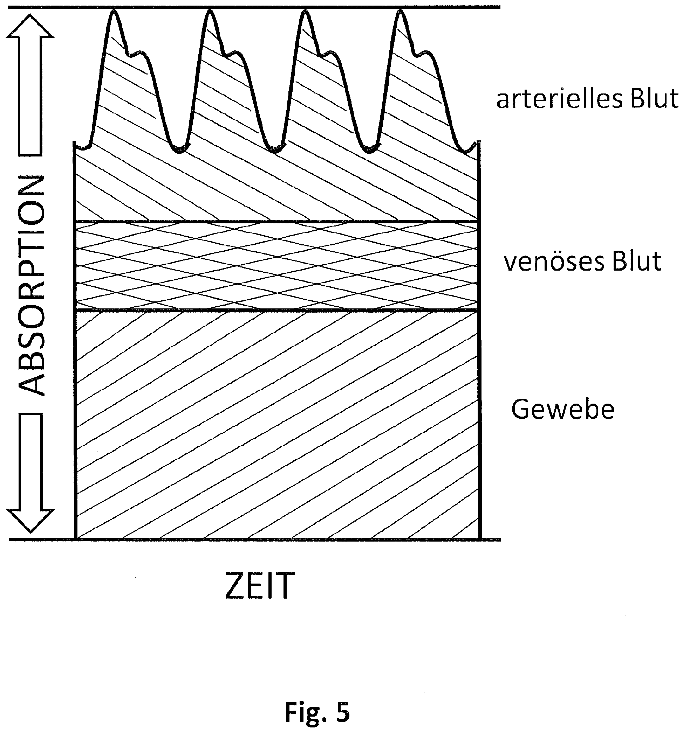

In Figur 1a ist eine erfindungsgemäße Vorrichtung 1 in Seitenansicht dargestellt. Die Vorrichtung 1 weist eine oder mehrere Beleuchtungseinrichtungen 20 (siehe Figur 4) auf, welche Messlicht 2 erzeugen. Die Beleuchtungseinrichtungen 20 dienen hierbei dazu, einen zu untersuchenden Messbereich 3, typischerweise einen Haut- und Gewebebereich, als im Wesentlichen zweidimensionalen Bereich bzw. Bereich mit relativ schmaler Erstreckung in Y-Richtung auszuleuchten. Der lineare Messbereich 3 wird somit in den verschiedenen Ausführungsformen jeweils durch die Beleuchtungseinrichtungen entweder reflexiv oder transmissiv beleuchtet und gibt Analyselicht 4 entsprechend seinem Transmissions- bzw. Reflexionsverhalten ab. Das Analyselicht 4 wird über einen Umlenkspiegel 5 in eine Spektrometereinheit 22 eingekoppelt. Das Analyselicht 4 liegt hierbei zur Bestimmung der Sauerstoffsättigung im Blut und weiteren Blutwerten im sichtbaren (VIS) und nahen Infrarotbereich (NIR), z. B. im Wellenlängenbereich zwischen 500 nm und 850 nm und weist eine Spektralverteilung entsprechend der Substanzzusammensetzung auf, wie mit Bezug zu Fig. 5 weiter unten erläutert wird. Somit enthält das Analyselicht 4 Spektren im relevanten Wellenlängenbereich zur Identifizierung der quantitativen Substanzzusammensetzung im Messbereich 3, also typischerweise der Substanzzusammensetzung des arteriellen Blutes und des Gewebes. Die gleiche Vorrichtung kann auch zum Bestimmen der Blutzuckerkonzentration verwendet werden, wenn der Wellenlängenbereich angepasst wird, typischerweise auf 800 nm bis 1200 nm.

Das Analyselicht 4 gelangt über einen Umlenkspiegel 5 und eine Abbildungsoptik 6 auf eine Blende 7. Die Abbildungsoptik 6 dient als Eingangsobjektiv für die Spektrometereinheit 22. Die Blende 7 ist länglich ausgebildet, vorzugsweise als Spalt oder Schlitz, z. B. mit einer Breite von typisch 10 $\mu$m bis 30 $\mu$m, und erstreckt sich in horizontaler Richtung bzw. z-Richtung (senkrecht zur Zeichenebene in Figur 1a). Falls in den Strahlengang weitere optische Elemente wie z. B. Filter oder weitere Spiegel eingesetzt sind, ist dies entsprechend zu berücksichtigen; erfindungsgemäß ist lediglich relevant, dass der Messbereich 3 so auf den Spalt der Blende 7 abgebildet wird, dass seine Erstreckung in z-Richtung der Spaltrichtung entspricht.

Der von der Blende 7 durchgelassene Streifen des Bildes des Messbereichs 3 wird als Licht über eine zweite Abbildungsoptik 8 auf ein Beugungsgitter 9 geworfen. Das Gitter ist für Blutwertmessungen im Monitoring typischerweise ein transmissives "Volume Phase Holographic"-Gitter mit einer Blazewellenlänge im Bereich 700 nm und ca 300-600 1/mm. Für Blutzuckermessungen ist das Gitter beispielsweise ein "Volume Phase Holographic Transmission Grating" mit 600 1/mm im Bereich von 900nm (Hersteller: Wasatch Photonics). Das Gitter 9 ist so aufgebaut und angeordnet, dass eine wellenlängendispersive Auffächerung des Analyselichts 4 senkrecht zur Richtung des Spaltes der Blende 7 erfolgt, d. h. in Querrichtung bzw. Y-Richtung; hier sind entsprechend auch abgewandelte Ausführungsformen möglich. Über eine dritte Abbildungsoptik 10 wird das gebeugte Licht als Beugungsbild auf eine Sensorfläche 11 eines Bildsensors 12 abgebildet. Auf der Sensorfläche 11 wird somit ein Beugungsbild der Blende 7 bzw. deren Spalt abgebildet, mit der Längserstreckung des Spalts (der z-Richtung) in einer Richtung und der wellenlängendispersiven Auffächerung des Beugungsbildes in der anderen Richtung. Der Bildsensor ist für Blutwertmessungen im Monitoring typischerweise ein CMOS-Kamerasensor des Typs Aptina MT9m032 (1,6 Mpixel) oder MT9P031 (5 Mpix).

Beispielsweise wird ein Sensor von Photonfocus (Typ A13121, 60dB) oder von Cypress (Typ IBIS5, 1.3 Megapixel, 64dB) verwendet.

In Figur 1b ist eine Draufsicht der Anordnung dargestellt.

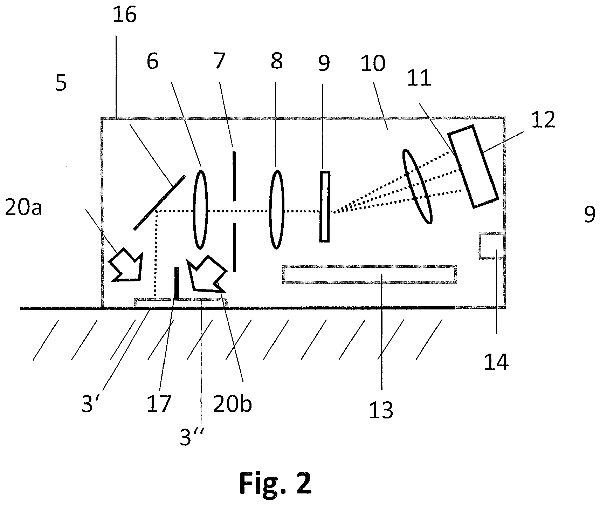

Figur 2 zeigt schematisch eine erfindungsgemässe Vorrichtung. Die verschiedenen Komponenten, insbesondere zwei Lichtquellen 20a / 20b, die vorstehend erwähnten Spiegel 5, Optiken 6, 8 und 10, die Blende 7 und das Beugungsgitter 9 sind in einem Gehäuse 16 angeordnet.

In dem Gehäuse 16 ist ausserdem eine Elektronikeinheit 13 vorhanden, welche einen Microcontroler (z. B. ein FX2-Baustein von Cypress) mit schneller serieller Datenumsetzung (z. B. USB2/ USB3) aufweist. Auch die LED-Konstantstromregelungen können hier untergebracht sein. Ein USB-Kabelanschluss 14 ermöglicht die serielle Datenübertragung und die Stromversorgung des Sensorkopfes.

Das Gehäuse 16 ist typischerweise ein Spritzgussteil aus einem Kunststoffmaterial. Bei der Verwendung von Linsen aus bekannten Miniaturobjektiven mit ca. 12 mm Gehäusedurchmesser kann eine Gehäusegrösse des Sensors von ca. 10 x 15 x 50 mm erreicht werden. Alternativ können auch direkt Objektive mit Gehäusedurchmessern von 8 mm eingesetzt werden. Das Gehäuse 16 weist eine Form auf, dank der es sich an der Messstelle befestigen lässt, z. B. am Ohrläppchen oder am Finger oder im Fall von Anwendungen in der Dialyse oder im nichtmedizinischen Bereich z. B. auch an Leitungen zum Transport des Messmediums. Ausserdem ist das Gehäuse zusätzlich mit dem Fachmann an sich bekannten Befestigungsmitteln versehen. Im Bereich des Lichtaustrittes bzw. Lichteintrittes ist das Gehäuse 16 mit einem antireflektiven Glasfenster verschlossen.

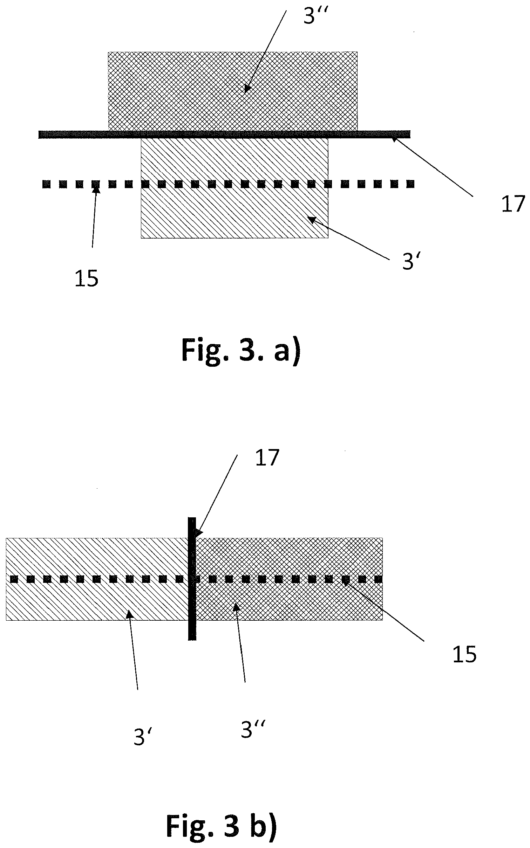

Eine Trennwand 17 unterteilt dabei zwei verschiedene Einstrahlbereiche 3', 3" zur Unterscheidung zwischen einer Reflexionsmessung (Einstrahlbereich 3') und einer Transmissionsmessung (Einstrahlbereich 3"), siehe dazu auch Figur 3b.

Durch die starke Änderung der Extinktionskoeffizienten in dem Spektralbereich ist es wichtig, die Aufnahmen sowohl in Reflexion als auch in Transflexion durchzuführen. Fig. 3a und 3b zeigen zwei Möglichkeiten auf, wie dies mit einem Sensorsystem erreicht werden kann.

In Fig. 3a ist eine zeitlich-räumliche Trennung vorgesehen. Dabei wird zuerst eine kurzzeitig gepulste Beleuchtung des Bereichs 3' (siehe Figur 2) für eine Reflexionsmessung durchgeführt. Die Spektrometereinheit 22 mit Eingangsobjektiv schaut hierbei auf die Abbildungslinie 15. Nachdem das Bild ausgelesen wurde, wird eine zweite gepulste Lichtquelle aktiviert, die den Bereich 3" auf der Haut ausleuchtet. Dabei kann Licht durch die Trennwand 17 nicht direkt zu der Aufnahmelinie 15 gelangen. Das Licht bewegt sich transflexiv durch das Gewebe, und ein Teil tritt hierbei bei der Aufnahmelinie 15 wieder aus, welcher dann für die transmissive Auswertung der Signale eingesetzt wird.

Bei der in Figur 3b dargestellten Möglichkeit wird das Licht nur im Bereich 3' eingestrahlt. Die 2D-Spektrometereinheit schaut aber über den gesamten Bereich 3' und 3" auf der Linie 15. Aufgrund der Ortsauflösung auf dem Array ist eine Differenzierung zwischen Licht aus den beiden Bereichen 3' und 3" möglich. Durch die Trennwand 17 wird in 3" nur ein transmittiertes Signal aufgenommen. Die zu erwartenden Intensitätsunterschiede auf dem Sensor der Bereiche 3' und 3" können durch einen fest eingesetzten Graufilter im Strahlengang hinter der Eintrittsblende 7 oder vor der Sensorfläche 11 ausgeglichen werden.

In Fig. 4 ist ein Blockdiagramm einer vorteilhaften Ausführung des Sensorsystems dargestellt. Die Beleuchtungseinheit 20 gibt Licht 2 auf den Messbereich 3. Dieses Licht wird wie bereits beschrieben in Reflexion oder Transflexion in nun veränderter Form als Analyselicht in die Spektrometereinheit 22 eingekoppelt. Nach einer spektralen Zerlegung in der Spektrometereinheit 22 wird aufgefächertes Licht 23 auf den Bildsensor 11 gegeben. Der Bildsensor 11 besteht aus einzelnen in einer Matrix angeordneten Photoelementen 24. Der Bildsensor 11 ist ein zweidimensionaler CMOS-Digitalkamera-Sensor: Wie in dem Blockdiagramm der Fig.4 angedeutet, weist er ein Pixelarray aus einzelnen, im VIS und VNIR-Spektralbereich sensitiven Pixeln auf, die in einer Matrixanordnung angeordnet sind. In der einen Richtung (beispielsweise der X-Richtung) werden aufgrund der Auffächerung des Lichts auf den einzelnen Pixeln Anteile des Lichtes in unterschiedlichen Wellenlängen abgebildet. Auf einer Sensorzeile in X-Richtung wird daher ein Spektrum des Analyselichts erfasst. In Y-Richtung liegen mehrere Messzeilen des Sensors parallel nebeneinander. Auf jeder dieser Zeilen wird ein Spektrum des Analyselichts gemessen. Indem eine Mehrzahl von parallelen, nebeneinander liegenden Zeilen, typischerweise 1000 Zeilen (oder bei 4 Mpix oder 5 MPix Sensoren typischerweise 2000 Zeilen), ausgelesen und aufaddiert werden, lässt sich erfindungsgemäss ein Signal mit verbessertem Signal-/Rauschverhältnis erzeugen.

In dem Sensor wird bereits die Verstärkung und Digitalisierung der photoelektrischen Signale vorgenommen. Diese werden dann über eine Verbindungsleitung 25 parallel oder seriell an einen Mikroprozessor 26 übertragen. Der Mikroprozessor 26 macht zum einen eine Umsetzung der Signale, zum anderen übernimmt er über eine Steuerleitung 28 die Steuerung der LED-Beleuchtungseinheit 20 und über eine Parametrierungsleitung 27 die Parametrierung des Bildsensors 11.

Ein derartiger CMOS-Bildsensor 11 ermöglicht es, mit einer einzigen Bildaufnahme gleichzeitig bis zu tausend Spektren, d. h. ein Spektrum pro Zeile, aufzunehmen mit einer Datentiefe von z. B. 12 Bit. Jedes der Spektren entspricht somit dem Spektrum eines Bildelementes der Blende, d. h. es entspricht einer Unterteilung der schlitzförmigen Blende 7 gemäss der Pixelanzahl des Sensors, die in Y-Richtung (siehe Figur 1a) aneinandergereiht sind.

Der Bildsensor 11 kann die Bildaufnahme z. B. mit einer Bildwiederholungsrate von z. B. 50 Aufnahmen pro Sekunde wiederholen. Da erfindungsgemäß beispielsweise für Monitoring-Anwendungen nur ein kleiner Spektralbereich im Bereich von 500 nm bis 850 nm oder für Blutzuckermessungen im Bereich von 800 nm bis 1200 nm relevant ist oder auch nur ein eingeschränkter Ortsbereich ausgelesen werden muss, kann die bei derartigen Bildsensoren 11 mögliche Teilbildaufnahme eingesetzt werden, so dass Teilbilder als "region of interest" (ROI) eingestellt werden, die es ermöglichen, nur den eingestellten interessierenden Bildbereich (entsprechend einem gewünschten Frequenzbereich) des Bildsensors 12 bei gleichzeitiger Beibehaltung der Grunddatenrate (Pixelrate) auszulesen, was die Anzahl der übertragenen frames, d. h. Bilder oder Teilbilder pro Sekunde erhöht.

Der Mikroprozessor 26 übernimmt ausserdem die Kommunikation über eine Kommunikationsleitung 29 mit einem Hauptprozessor 30 des Systems. Dabei werden die Bilddaten zum Hauptprozessor 30 übertragen, und der Hauptprozessor gibt die Parametrierungsdaten an das Sensorsystem 33. Über die Kommunikationsleitung 29 findet auch die Stromversorgung des Sensorsystems 33 statt. Eine vorteilhafte Ausführung der Verbindung ist eine USB-Verbindung, welche gleichzeitig eine hohe Datenübertragungsrate und eine Spannungsversorgung mit 5 Volt ermöglicht. Der Hauptprozessor 30 ist mit einer Anzeige- und Eingabeeinheit 31 versehen, mit der die systemrelevanten Parameter von einem Bediener eingestellt und die aktuell ermittelten Daten dargestellt werden können. Prozessor, Speicher und Benutzerterminal können in einer Einheit 32 untergebracht werden, die vom Patienten entfernt aufgestellt werden kann. Typischerweise ist der Hauptprozessor ein Zweikern-Rechner, wobei im ersten Kern Bildbearbeitung und im zweiten Kern die Auswertung der Daten zur Bestimmung der Gewebe- und Blutwerte erfolgt.

In Fig. 5 wird schematisch der typische Zeitverlauf der Signale bei der in vivo Blutmessung dargestellt. Das optische Signal weist hierbei einen konstanten Anteil und einen pulsatilen Anteil auf. Der konstante Anteil, oder präziser ausgedrückt der sich nur langfristig ändernde Anteil, kommt zum einen vom venösen Blut, zum anderen vom Gewebe. Dabei ist das Signal vom Gewebe weiterhin in zwei Bereiche zu unterscheiden. Ein Anteil ist von den Inhaltsstoffen des Gewebes abhängig, der andere Anteil hängt von den Streueigenschaften des Gewebes ab, welche die realen Lichtwege beeinflussen. Der pulsatile Anteil wird durch das Pumpen des arteriellen Blutes vom Herz erzeugt. Die Absorption im arteriellen Blut ist bei der Systole und der Diastole nicht gleich. Dies erlaubt eine Differenzierung. Hierbei wird stark sauerstoffgesättigtes Blut in den gemessenen Körperteil gepumpt. Hierbei ist die Sauerstoffsättigung des Hämoglobins bei einem gesunden Menschen im Bereich von 95% bis 99%. Der pulsierende Signalanteil ist wellenlängenabhängig und hängt von der Messstelle und der Messart (reflexiv/transmissiv) ab. Ausserdem spielt es eine Rolle, ob die Messstelle erwärmt wird. Der pulsatile Anteil bei Fingermessungen bei gesunden Personen kann (bei transmissiven Messungen) 3% bis 20% betragen. Bei reflexiven Messungen z. B. am Ohrläppchen kann der Wert 0,5% bis 1,5% betragen. Bei Patienten mit schlechter Durchblutung oder akuten Problemen ist der pulsierende Anteil noch niedriger.

Aus diesem niedrigen Anteil müssen Puls und Sauerstoffgehalt oder der Blutzuckergehalt bestimmt werden. Erfindungsgemäss können Spektren sehr schnell (ca. 50-100 Hz) aufgenommen werden, und bei jeder Aufnahme können ca. 1000 Einzelspektren zu einem Spektrum aufaddiert werden. Damit kann man in der Spektroskopie den konstanten und den pulsierenden Anteil im Spektrum trennen.

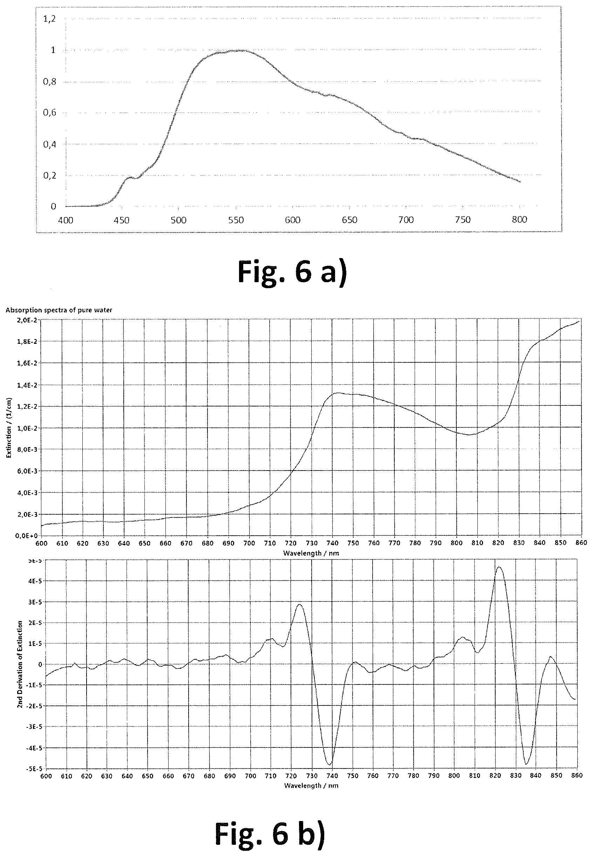

In Figur 6a wird das Spektrum einer typischen LED-Beleuchtungseinheit für Monitoring-Anwendungen dargestellt. Für das System wird eine Beleuchtungseinheit benötigt, welche Licht im Spektralbereich von 500 nm bis 850 nm zur Verfügung stellt.

Gezeigt ist hier das Spektrum einer geeigneten breitbandigen LED, welche wie eine Weisslicht-LED einen blauen Emitter (450nm) zur Anregung eines Farbstoffes hat. Diese LED weist vor allem im Spektralbereich 500 nm bis 650 nm eine gute Intensitätsverteilung auf. Je nach Anwendungsgebiet könnte auch auf eine Weißlicht-LED mit substantiell niedriger Farbtemperatur zurückgegriffen werden, es könnten andere Farbstoffzusammensetzungen eingesetzt werden oder es könnten zusätzlich monochrome LEDs zur Beleuchtung hinzugefügt werden.

In Figur 6b wird die Absorptionskurve des Wassers und deren zweite Ableitung gezeigt. Wasser hat bei 730 nm und 830nm eine schwache Absorptionsbande (Kombinationsschwingung $av_1 + bv_3$; mit $a + b = 4$). Diese ist mit der hier vorgestellten Technik aber gut auswertbar. Da aus der Literatur bekannt ist, dass der Wasseranteil im Blut beim Menschen sehr konstant ist und zwischen 80 und 85 Volumenprozent liegt, ist es möglich, im pulsatilen Anteil eine Absolutbestimmung der Konzentrationen auch über das Wassersignal vorzunehmen. Dies kann beim Monitoring genauso angewendet werden wie beispielsweise bei der Blutzuckermessung.

Für Blutzuckermessungen wird hingegen eine Beleuchtungseinheit benötigt, welche Licht im Spektralbereich von 800 nm bis 1200 nm zur Verfügung stellt (nicht gezeigt).

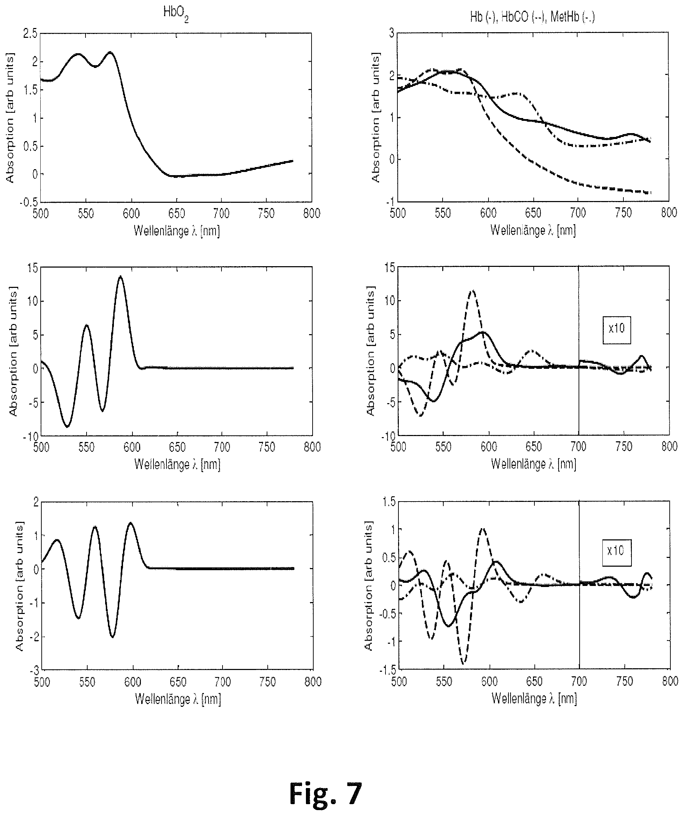

In Fig. 7 sind relevante Spektren von oxigeniertem Hämoglobin (HbO2) und weiteren Hämoglobinderivaten gezeigt als Absorptionsspektrum, als erste und als zweite Ableitung, wobei auf der Abszisse die Wellenlänge λ von 500 nm - 800 nm aufgetragen ist. Diese sehr präzise bekannten Daten ermöglichen mit den bereits beschriebenen multivariaten Regressionen die Berechnung der Anteile der Substanzen.

Wichtig hierbei ist, dass mit der vorgestellten Technik nicht nur eine Auswertung der Absorptionssignale, sondern über die Nutzung der zweiten Ableitung eine sehr präzise Abgrenzung zu den anderen Substanzen durchgeführt werden kann. Zur Auswertung wird gegebenenfalls ein multivariates statistisches Analyseverfahren verwendet. Vorteilhafterweise werden Einzelspektren aller relevanten und zu erkennenden Substanzen vorab gemessen und gespeichert.

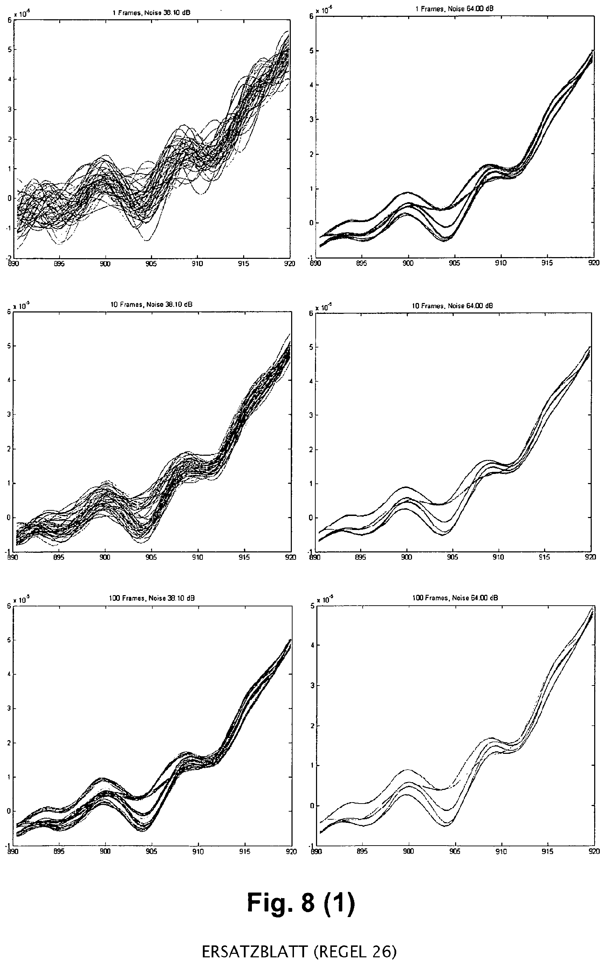

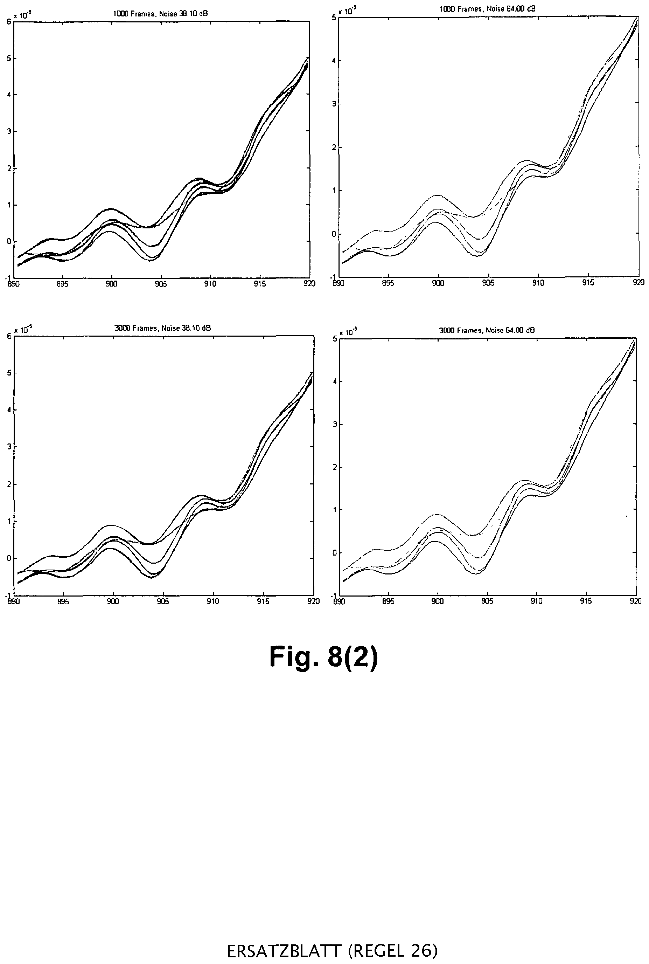

Die Figuren 8 bis 11 zeigen einen konkreten Aufbau eines Sensors und damit gemachte Messungen.

In Figur 8 sind Simulationen von verschiedenen Spektren dargestellt. Die Spektren in der linken Spalte wurden auf der Basis eines ersten Typs eines Sensors (mit 38,10 dB) erzeugt. Die Spektren in der rechten Spalte wurden auf der Basis eines zweiten Typs eines Sensors (mit 64 dB) erzeugt. Ein solcher Sensor ist z.B. der 1.3 Megapixel CMOS Image Sensor IBIS5-B-1300 von Cypress mit 1280x1024 Pixeln mit einer Pixelgrösse von $6.7\,\mu m$ x $6.7\,\mu m$. Zur Simulation werden 5 sehr gut gemessene Spektren als Ausgangspunkt genommen. Aus diesen werden jeweils 30 Einzelspektren erzeugt, die mit einem künstlichen Sensorrauschen überlagert werden. Das Ergebnis wird geplottet. Es zeigt, welche Streuungen man bei Einzelmessungen zu erwarten hätte.

Von oben nach unten zeigen die verschiedenen Darstellungen den Einfluss der Anzahl der Spektrenintegrationen. Die Darstellungen zeigen die zweite Ableitung des an einem menschlichen Finger erzeugten Spektrums im Wellenlängenbereich von 890 bis 920 nm. Von oben nach unten zeigen die einzelnen Darstellungen die gleiche Anzahl (simulierter) Spektren, die durch die Addierung von einer nach unten zunehmenden Zahl von Einzelspektren erzeugt wurden. In einem "Frame" sind dabei bereits 1000 Zeilen aufintegriert. Die oberste Darstellung zeigt 1 Frame. Die unterste Darstellung zeigt 3000 Frames.

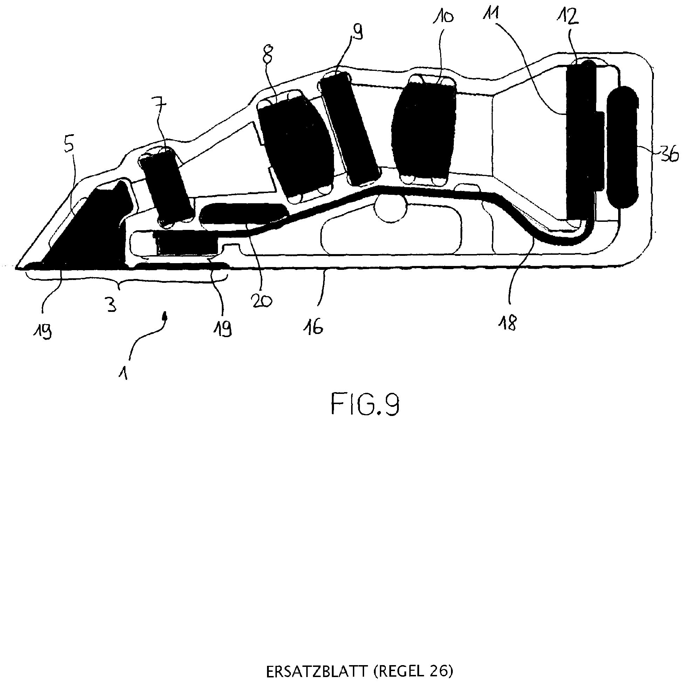

Figur 9 zeigt eine nicht-erfindungsgemässe Vorrichtung 1. Gleiche Bezugzeichen bezeichnen in dieser Figur die gleichen Bauteile wie in den vorhergehenden Figuren. Die Vorrichtung 1 weist ein Gehäuse auf, in dem die verschiedenen optischen und elektronischen Komponenten angeordnet sind. Die Messung erfolgt an einem Finger. Der Finger wird in den Messbereich 3 geführt. Dieser Sensor ist insbesondere zur Messung von Blutzucker geeignet. Durch eine breitbandige LED 20 wird typischerweise Licht im Spektralbereich von 800 bis 1200 nm gegen den Messbereich 3 ausgestrahlt. Das Gehäuse 16 weist eine Öffnung zum Austritt des Lichtes auf. Die Öffnung kann mit einer für das austretende Licht transparenten Abdeckung 19 versehen sein. Das durch den Finger wieder austretende Licht wird durch eine zweite Öffnung im Gehäuse 16, welche ebenfalls mit einer für das Licht transparenten Abdeckung 19 versehen ist, in das Gehäuse 16 geleitet. Über eine Spiegelanordnung 5, eine Spaltblende 7 und eine erste Abbildungsoptik 8 wird das Licht auf ein Beugungsgitter 9 gelenkt. Durch das Beugungsgitter 9 wird das Licht wellenlängenabhängig aufgefächert und über eine zweite Abbildungsoptik 10 auf die Sensorfläche 11 des Bildsensors 12 geleitet. Der Bildsensor 12 und die LED 20 sind auf einer gemeinsamen Leiterplatte 18 im Gehäuse 16 angeordnet. Die Leiterplatte 18 ist ausserdem mit Elektronikkomponenten zur Steuerung der LED 20 und des Bildsensors 12 versehen. Insbesondere weist die Leiterplatte 18 auch einen USB-Controller 36 und nicht genauer dargestellte USB Anschlüsse auf. Diese USB-Schnittstelle erlaubt einerseits Energiezufuhr zu der Vorrichtung 1. Andererseits ermöglicht sie Datenaustausch mit einem externen Rechner oder Anzeigegerät. Auf dem Sensor werden typischerweise 4x4 Pixel zusammengefasst (Binning). Die derart zusammengefassten Daten werden über die USB-Schnittstelle an einen Rechner übergeben. Dort werden die Spektren nach Herausrechnung der statischen optischen Verzerrung des Sensors aufaddiert.

Als LED wird eine High Power LED verwendet. Als Linsen werden geeignete Achromate verwendet. Das Gitter ist ein je nach Spektralbereich optimiertes Gitter mit typischerweise 300 l/mm oder 600 l/mm.

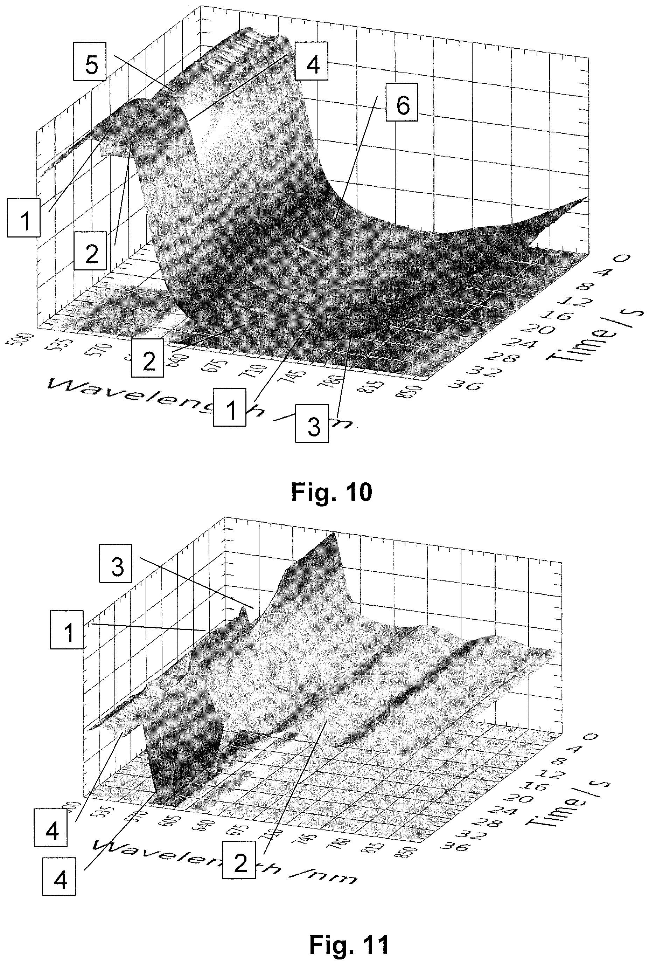

Figur 10 zeigt ein mit dem Sensor gemäss Figur 9 erfasstes Spektrum im zeitlichen Verlauf. Das Spektrum ist im Spektralbereich von 500 bis 850 nm in Transflexion (an einer Stelle ins Gewebe eingekoppelt und an einer anderen Stelle auf der gleichen Seite wieder ausgekoppelt) an einem Finger gemessen. Figur 10 zeigt die Spektren im zeitlichen Verlauf mit zwei unterschiedlichen Anpressdrücken (geringerer Anpressdruck 0 bis ca. 10sec und ab 20sec/ höherer Anpressdruck ca. 10 bis 20 s). Im Spektralbereich von 520 bis 580 nm ist der Puls aufgrund der starken Absorption des oxidierten Hb gut erkennbar. Ebenfalls ist im Bereich von 650 bis 850 nm der Puls deutlich zu erkennen, da das Licht tiefer in den Finger eindringt und dort mehr arterielles Blut zum Signal beiträgt. Diese beiden Regionen sind in Figur 10 mit der Ziffer 1 gekennzeichnet. Bei stärkerem Anpressdruck zwischen 10 und 20 s ist der Puls hingegen deutlich weniger ausgeprägt. Vor allem beim Monitoring, wo eine pulsaufgelöste Messung wichtig ist, ist also darauf zu achten, dass kein allzu grosser Anpressdruck besteht.

Bei den Ziffern 2 sind charakteristische Bandenformen des oxigenierten Hämoglobins (HbO2-arterielles Blut) zu erkennen. Bei 540/578 nm besteht ein Doppelpeak. Im Bereich von 660 bis 680 nm ist die Absorption sehr gering.

Die Ziffer 3 in Figur 10 zeigt eine Absorptionsbande des deoxigenierten Hämoglobins bei 760 nm.

Bei niedrigeren Wellenlängen nimmt die Absorption bei höherem Anpressdruck (Ziffer 4 in Figur 10) stark ab. Die Absorption wird kleiner, weil verhältnismässig weniger Blut zur Verfügung steht (das Blut wird aus dem Finger gedrückt).

Verschiebungen der Absorptionsdoppelbande (durch die Ziffer 5 gekennzeichnet) geben Aufschluss über den Gehalt an HbCO im Blut.

Der mit 6 gekennzeichnete Bereich bei 650 nm dient zur Identifikation des Methämoglobingehalts (MetHB).

Figur 11 zeigt die zweite Ableitung der in Figur 10 gezeigten Spektren. Es ist an sich bekannt, mit der zweiten Ableitung der Spektren zu arbeiten (Derivativspektroskopie). Damit können alle konstanten Anteile aus dem Spektrum entfernt werden. Dadurch können Artefakte aufgrund von Beleuchtungsschwankungen, aber auch unterschiedliche Lichtlevel aufgrund der Gewebestreuung beseitigt werden. Ausserdem werden die signifikanten Informationen verstärkt. Vor allem ist die Verwendung von Ableitungen dann von Nutzen, wenn sich die Absorptionsmaxima von Komponenten in Mischungen aus mehreren Substanzen nur geringfügig unterscheiden oder überlagern. Die zweite Ableitung gibt viele zusätzliche Informationen. Der Puls ist auch in der zweiten Ableitung noch zu erkennen. Er tritt aber nur dort stark hervor, wo sich die Absorption zwischen dem arteriellen Blut (HbO2) und dem Gewebe/venösen Blut stark unterscheidet. Dies ist insbesondere im Spektralbereich zwischen 600 und 630 nm der Fall (mit Ziffer 1 gekennzeichnet). Im längerwelligen Spektralbereich unterscheiden sich die Absorptionen weniger stark. Die Puls-Amplitude der zweiten Ableitung ist daher deutlich geringer (siehe Ziffer 2 im mittleren Bereich in Figur 11).

Auch in der zweiten Ableitung ist bei starkem Anpressdruck der Puls schwächer erkennbar. Bei der Ziffer 3 verändert sich nicht nur die Puls-Amplitude, sondern auch die Form und die Position der Absorptionsbanden, wenn der Druck erhöht wird. Dies zeigt, dass durch Erhöhung des Drucks insbesondere arterielles Blut aus dem Finger herausgedrückt wird.

Der Doppelpeak des oxigenierten Hämoglobins ist (mit Ziffer 4 gekennzeichnet) in der zweiten Ableitung sehr deutlich erkennbar.

Im Gewebe und im venösen Blut ist der Anteil an deoxigeniertem Hb (HHb) grösser. Die dafür signifikante Absorptionsbande liegt bei 760 nm. Sie ändert sich in Abhängigkeit des Anpressdrucks kaum, da arterielles Blut fast vollkommen oxigeniert ist. Aus dem Verhältnis zwischen der Intensität dieser Bande und dem oxigenierten Hämoglobin (erkennbar durch den Doppelpeak im Bereich 4) kann die Gewebesauerstoffsättigung (StiO2) bestimmt werden.

Aus der zweiten Ableitung und insbesondere aus deren Kurvenverlauf und den Positionen der einzelnen Absorptionspeaks lässt sich die Konzentration von HbCO bestimmen. Aufgrund der bekannten, wellenlängenabhängigen Absorptionskoeffizienten von HbO2, HHb, HbCO und HbMet können die Konzentrationen bestimmt werden.

Zur Analyse der Blutinhaltsstoffe bzw. deren Veränderung ist es nützlich, die Daten zeitaufgelöst (und damit pulsaufgelöst) zu erfassen. Dies ermöglicht es, gezielt Daten aus der Systole und der Diastole getrennt zu erfassen und auch getrennt zu analysieren (siehe auch Darstellung in Figur 4, wo hohe Absorptionen auf die Systole und verhältnismässig niedrige Absorptionen im arteriellen Blut auf die Diastole hinweisen). Insbesondere ergibt die Differenz zwischen den Spektren der Diastole und der Systole das reine Spektrum des arteriellen Blutanteils. Wie Figur 5 zeigt, ist die Differenz der Absorption zwischen Systole und Diastole aber im Vergleich zur Gesamtabsorption verhältnismässig gering. Aufgrund der insgesamt grossen Absorption sind auf dem Sensor nur noch geringe Lichtsignale vorhanden. Weil erfindungsgemäss gleichzeitig die Spektren von mehreren nebeneinander liegenden Zeilen eines zweidimensionalen Sensors aufgenommen und aufaddiert werden, wird ein Signal mit ausreichend guter Qualität erzielt. Daher können auch sehr geringe Differenzspektren zwischen Systole und Diastole ausgewertet werden.

## Patentansprüche

1. Vorrichtung zum Erkennen und Überwachen von physiologischen Blutwerten eines Lebewesens für eine nichtinvasive in vivo Messung, enthaltend
wenigstens eine Lichtquelle (20; 20a, 20b) zum Erzeugen von breitbandigem Licht (2; 2a, 2b), bevorzugt wenigstens umfassend 500 nm bis 850 nm und/oder 800 nm bis 1200 nm, insbesondere eine LED, zum Beaufschlagen eines Messbereichs (3; 3', 3"),
Mittel zum Auffächern des von wenigstens einer Messstelle (3; 3', 3") zurückgeworfenen oder durch die Messstelle hindurchtretenden Analyselichts (4) entsprechend den Wellenlängen des Analyselichts (4),
einen zweidimensionalen Sensor-Array (11) zur Aufnahme des aufgefächerten Analyselichts (13), der derart angeordnet ist, dass Licht mit unterschiedlichen Wellenlängen an unterschiedlichen Stellen des Sensor-Arrays (11) auftrifft, wobei die Vorrichtung (1) ein Gehäuse (16) aufweist und als kompakte Baueinheit ausgebildet ist, welche wenigstens die Lichtquelle (20; 20a, 20b), die Mittel zum Auffächern (9) und den Sensor-Array (11) enthält, und welche kleiner als 20mm x 30mm x 100mm, bevorzugt von circa 10mm x 15mm x 50mm, ausgebildet ist, wobei das Gehäuse zusätzlich mit Befestigungsmitteln versehen ist, derart, dass die Vorrichtung direkt an einem Patienten befestigbar ist, wobei der zweidimensionale Sensorarray (11) derart angeordnet ist, dass das aufgefächerte Analyselicht auf eine Mehrzahl von nebeneinander liegenden Zeilen des zweidimensionalen Sensorarrays (11) auftrifft, wobei aufgrund der wellenlängenabhängigen Auffächerung des Messlichts das Messlicht auf einer Zeile des Sensors abgebildet wird und das das Spektrum gleichzeitig von mehreren parallel nebeneinander liegenden Zeilen des Sensorarrays erfasst wird,
wobei die Vorrichtung (1) eine Rechneranordnung aufweist, die zum Addieren der durch die nebeneinander liegenden Zeilen erzeugten Spektren ausgebildet ist,
wobei die Vorrichtung (1) eine Spaltblende (7) aufweist, die zwischen einem Eintrittsbereich für das Analyselicht (4) und den Mitteln (9) zum Auffächern angeordnet ist und die Spaltblende (7) bezogen auf die Mittel (9) zum Auffächern des Lichtes so angeordnet ist, dass ein längliches Bild in einer zum länglichen Bild verschiedenen Richtung, vorzugsweise senkrecht dazu, aufgefächert wird,
und wobei der zweidimensionalen Sensor-Array (11) ein CMOS Bildsensor mit einer Bildrate von über 100Hz ist.

2. Vorrichtung nach Anspruch 1, **dadurch gekennzeichnet, dass** das Gehäuse (16) zum Anbringen an einer Körperstelle eines menschlichen Patienten, insbesondere am Finger oder an einem Ohrläppchen, ausgebildet ist.

3. Vorrichtung nach einem der Ansprüche 1 oder 2, **dadurch gekennzeichnet, dass** die Mittel zum Dispergieren ein Beugungsgitter (9), insbesondere ein holografisches Gitter aufweisen.

4. Vorrichtung nach einem der Ansprüche 1 bis 3, **dadurch gekennzeichnet, dass** die Vorrichtung einen A/D-Wandler aufweist.

5. Vorrichtung nach einem der Ansprüche 1 bis 4, **dadurch gekennzeichnet, dass** die Vorrichtung einen Verstärker aufweist, der vorzugsweise parametrierbar ist.

6. Vorrichtung nach einem der Ansprüche 1 bis 5, **dadurch gekennzeichnet, dass** die Vorrichtung einen Anschluss für eine elektrische Kommunikationsverbindung (29) aufweist und dass die Vorrichtung insbesondere keine Anschlüsse für Zu- oder Wegleitung von Licht aufweist.

7. Vorrichtung nach einem der Ansprüche 1 bis 6, **dadurch gekennzeichnet, dass** die Vorrichtung zur Transmissionsmessung, wobei das Analyselicht an einer anderen Stelle als der Messbereich nach Transmission durch Gewebe wieder abgegeben wird, und zur Reflexionsmessung, wobei das Analyselichts direkt vom Messbereich reflektiertes Licht ist, ausgebildet ist.

8. Vorrichtung nach Anspruch 7, **dadurch gekennzeichnet, dass** die Vorrichtung eine Rechneranordnung (26) aufweist, welche so ausgebildet ist, dass alternierend eine Transmissions- und eine Reflexionsmessung durchführbar ist, wobei die Vorrichtung eine erste Lichtquelle zur Durchführung einer Reflexionsmessung durch Beleuchten eines ersten Messbereichs (3') und eine zweite Lichtquelle (20b) zum Beleuchten eines zweiten Messbereichs (3") zur Transmissionsmessung aufweist.

9. Vorrichtung nach Anspruch 8, **dadurch gekennzeichnet, dass** die Vorrichtung mit Mitteln (17) zum Trennen des Analyselichts (4) aus einem Reflexionsbereich (3') und einem Transmissionsbereich (3") versehen ist.

10. Vorrichtung nach einem der Ansprüche 1 bis 9, **dadurch gekennzeichnet, dass** die Vorrichtung zur Abtastung mit einer Frequenz > 50 Hz ausgebildet ist, wobei ein Gewebeanteil und ein pulsatiler Anteil der gemessenen physiologischen Blutwerte ermittelbar ist.

11. Vorrichtung nach einem der Ansprüche 1 bis 10, **dadurch gekennzeichnet, dass** die Vorrichtung eine Rechneranordnung (26) aufweist, welche derart ausgebildet ist, dass Messungen zeitaufgelöst durchgeführt werden.

12. Vorrichtung nach einem der Ansprüche 1 bis 11, **dadurch gekennzeichnet, dass** die Vorrichtung eine Rechneranordnung (26) aufweist, welche derart ausgebildet ist, dass eine zweite Ableitung der erfassten Spektren ermittelt wird und dass physiologische Blutwerte auf der Basis dieser zweiten Ableitung ermittelt werden.

13. Kombination aus einer externen Rechneranordnung (32) und einer Vorrichtung nach einem Ansprüche 1 bis 10, wobei die Vorrichtung (1) und die Rechneranordnung (32) über ein Kommunikationskabel (29) miteinander verbunden oder verbindbar sind, wobei die Rechneranordnung derart ausgebildet ist, dass Messungen zeitaufgelöst durchgeführt werden oder die Rechneranordnung derart ausgebildet ist, dass eine zweite Ableitung der erfassten Spektren ermittelt wird und dass physiologische Blutwerte auf der Basis dieser zweiten Ableitung ermittelt werden.

14. Verfahren zum Erkennen und Überwachen von physiologischen Blutwerten eines Lebewesens in einer nichtinvasiven in vivo Messung, bestehend aus den folgenden Schritten, mit einer Vorrichtung nach einem der Ansprüche 1 bis 12

 - Beaufschlagen wenigstens des Messbereichs (3; 3', 3") mit Licht (2; 2a, 2b) aus der breitbandigen Lichtquelle (20; 20a, 20b) zum Erzeugen von breitbandigem Licht (2; 2a, 2b), bevorzugt wenigstens umfassend 500 nm bis 850 nm und/oder 800 nm bis 1200 nm, insbesondere eine LED,
 - Erfassen von in Reflexion und/oder Transmission zurückgeworfenem Analyselicht (4) mit dem CMOS Sensor-Array (11), wobei
 - wellenabhängiges Auffächern des erfassten Analyselichts (4) mit den Mitteln zum Auffächern und Abbilden der einzelnen, wellenlängenabhängigen Anteile des erfassten Analyselichts (4) auf den zweidimensionalen CMOS Sensor-Array (11), wobei das aufgefächerte Analyselicht, nämlich das Spektrum, auf einer Mehrzahl von nebeneinander liegenden Zeilen des Sensorarrays (11) abgebildet wird und die durch die einzelnen Zeilen erzeugten Spektren addiert werden,
 - Auswerten des derart erzeugten Spektrums mittels der Rechneranordnung, die zum Addieren der durch die nebeneinander liegenden Zeilen erzeugten Spektren ausgebildet ist, zum Ermitteln der Blutwerte,

wobei vorgängig das Gehäuse an einer Messstelle (3; 3', 3") eines Lebewesens mit den Befestigungsmitteln befestigt wird, welches Gehäuse die Lichtquelle und den Sensor-Array enthält.

**15.** Verfahren nach Anspruch 14, **dadurch gekennzeichnet, dass** das Analyselicht (4) an einem Beugungsgitter (9) aufgefächert wird.

**16.** Verfahren nach Anspruch 14 oder 15, **dadurch gekennzeichnet, dass** das Analyselicht (4) in Reflexion, wobei es sich beim Analyselicht um direkt vom Messbereich reflektiertes Licht handelt, und in Transmission, wobei es sich beim Analyselicht um an einer andern Stelle nach Transmission durch Gewebe wieder abgegebenes Analyselicht handelt, erfasst wird.

**17.** Verfahren nach einem der Ansprüche 14 bis 16, **dadurch gekennzeichnet, dass** das Analyselicht (4) zeitaufgelöst mittels der Rechneranordnung ausgewertet wird.

**18.** Verfahren nach einem der Ansprüche 14 bis 17, **dadurch gekennzeichnet, dass** zur Ermittlung der physiologischen Blutwerte in einer Rechneranordnung die zweite Ableitung der erfassten Spektren ermittelt wird.

**19.** Verfahren nach einem der Ansprüche 14 bis 18, **dadurch gekennzeichnet, dass** eine Abtastung mit einer Frequenz von mehr als 50 Hz vorgenommen wird und dass ein Gewebeanteil und ein pulsatiler Anteil der gemessenen physiologischen Blutwerte mittels der Rechneranordnung ermittelt werden.

**Claims**

**1.** Device for identifying and monitoring physiological blood values of a living being for a non-invasive in vivo measurement, containing at least one light source (20; 20a, 20b) for generating broadband light (2; 2a, 2b), preferably at least comprising 500 nm to 850 nm and/or 800 nm to 1200 nm, more particularly an LED, for acting on a measurement region (3; 3', 3"),
means for spreading the analysis light (4) according to the wavelength thereof, which analysis light (4) has been returned by at least one measurement point (3; 3', 3") or has passed through the measurement point,
a two-dimensional sensor array (11) for recording the spread analysis light (13), which sensor array is arranged such that light with different wavelengths impinges on different points of the sensor array (11), with the device (1) having a housing (16) and being designed as compact assembly which contains at least the light source (20; 20a, 20b), the means for spreading (9) and the sensor array (11) and which is designed to be smaller than 20 mm x 30 mm x 100 mm, preferably approximately 10 mm x 15 mm x 50 mm, with the housing additionally being provided with attachment means, in such a way that the device is able to be attached directly to a patient, with the two-dimensional sensor array (11) being arranged such that the spread analysis light impinges on a plurality of adjacent rows of the two-dimensional sensor array (11), with the measurement light being imaged onto a row of the sensor on account of the wavelength-dependent spread of measurement light and the spectrum being captured simultaneously by a plurality of rows of the sensor array lying parallel next to one another,
with the device (1) having a computer arrangement which is designed to add the spectra generated by the adjacent rows,
with the device (1) having a slit aperture (7), which is arranged between an inlet region for the analysis light (4) and the means (9) for spreading, and, with respect to the means (9) for spreading the light, the slit aperture (7) being arranged such that an elongate image is spread open in a direction different from, preferably perpendicular to, the elongate image,
and with the two-dimensional sensor array (11) being a CMOS image sensor with a frame rate of more than 100 Hz.

**2.** Device according to Claim 1, **characterized in that** the housing (16) is designed to be affixed to a point on the body of a human patient, more particularly to the finger or an earlobe.

**3.** Device according to either of Claims 1 and 2, **characterized in that** the means for dispersing have a diffraction grating (9), more particularly a holographic grating.

**4.** Device according to any one of Claims 1 to 3, **characterized in that** the device has an analogue/digital converter.

**5.** Device according to any one of Claims 1 to 4, **characterized in that** the device has an amplifier, which is preferably able to be parameterized.

**6.** Device according to any one of Claims 1 to 5, **characterized in that** the device has a connector for an electrical communication connection (29) and **in that** the device more particularly has no connectors for guiding light thereto or away therefrom.

7. Device according to any one of Claims 1 to 6, **characterized in that** that the device is designed to measure transmission, with the analysis light being re-emitted at a different point to the measurement region following the transmission through the tissue, and to measure reflection, with the analysis light being light that has been directly reflected by the measurement region.

8. Device according to Claim 7, **characterized in that** the device has a computer arrangement (26), which is designed such that it is possible to alternately carry out a transmission measurement and a reflection measurement, with the device having a first light source for carrying out a reflection measurement by illuminating a first measurement region (3') and a second light source (20b) for illuminating a second measurement region (3") for carrying out a transmission measurement.

9. Device according to Claim 8, **characterized in that** the device is provided with means (17) for separating the analysis light (4) from a reflection region (3') and a transmission region (3").

10. Device according to any one of Claims 1 to 9, **characterized in that** the device is designed for sampling with a frequency > 50 Hz, with it being possible to establish a tissue component and a pulsatile component of the measured physiological blood values.

11. Device according to any one of Claims 1 to 10, **characterized in that** the device has a computer arrangement (26), which is designed such that the measurements are carried out in a time-resolved fashion.

12. Device according to any one of Claims 1 to 11, **characterized in that** the device has a computer arrangement (26), which is designed such that a second derivative of the captured spectra is established and that physiological blood values are established on the basis of this second derivative.

13. Combination of an external computer arrangement (32) and a device according to any one of Claims 1 to 10, wherein the device (1) and the computer arrangement (32) are interconnected or are able to be interconnected by means of a communication cable (29), wherein the computer arrangement is designed such that measurements are carried out in a time-resolved fashion or the computer arrangement is designed such that a second derivative of the captured spectra is established and that physiological blood values are established on the basis of this second derivative.

14. Method for identifying and monitoring physiological blood values of a living being in a non-invasive in vivo measurement, consisting of the following steps, in conjunction with a device according to any one of Claims 1 to 12:

- applying light (2; 2a, 2b) from the broadband light source (20; 20a, 20b) for generating broadband light (2; 2a, 2b), preferably at least comprising 500 nm to 850 nm and/or 800 nm to 1200 nm, more particularly an LED, onto at least the measurement region (3; 3', 3"),
- capturing analysis light (4) returned in reflection and/or transmission mode using the CMOS sensor array (11), wherein
- carrying out wave-dependent spreading of the captured analysis light (4) using the means for spreading, and imaging the individual, wavelength-dependent components of the captured analysis light (4) on the two-dimensional CMOS sensor array (11), the spread analysis light, specifically the spectrum, being imaged on a plurality of rows of the sensor array (11) lying next to one another and the spectra generated by the individual rows being added,
- evaluating the spectrum generated thus by means of the computer arrangement which is designed to add the spectra generated by the rows lying next to one another, for establishing a blood values,

wherein the housing is attached in advance to a measurement point (3; 3', 3") of a living being using the attachment means, said housing containing the light source and the sensor array.

15. Method according to Claim 14, **characterized in that** the analysis light (4) is spread at a diffraction grating (9).

16. Method according to Claim 14 or 15, **characterized in that** the analysis light (4) is captured in a reflection mode, with the analysis light being light reflected directly by the measurement region, and in a transmission mode, with the analysis light being analysis light re-emitted at a different point following transmission through the tissue.

**17.** Method according to any one of Claims 14 to 16, **characterized in that** the analysis light (4) is evaluated in a time-resolved fashion by means of the computer arrangement.

**18.** Method according to any one of Claims 14 to 17, **characterized in that** the second derivative of the captured spectra is established in a computer arrangement for establishing the physiological blood values.

**19.** Method according to any one of Claims 14 to 18, **characterized in that** sampling is undertaken at a frequency of greater than 50 Hz and **in that** a tissue component and a pulsatile component of the measured physiological blood values are established by means of the computer arrangement.

**Revendications**

**1.** Dispositif permettant d'identifier et de surveiller des valeurs sanguines physiologiques d'un être vivant pour une mesure in vivo non invasive, comprenant
au moins une source lumineuse (20 ; 20a, 20b) pour générer de la lumière à large bande (2 ; 2a, 2b), comprenant de préférence au moins de 500 nm à 850 nm et/ou de 800 nm à 1200 nm, en particulier une LED, pour l'application à une zone de mesure (3 ; 3', 3"),
des moyens pour disperser la lumière d'analyse (4) renvoyée par au moins un point de mesure (3 ; 3', 3") ou traversant le point de mesure selon les longueurs d'onde de la lumière d'analyse (4),
un réseau de capteurs bidimensionnel (11) pour recevoir la lumière d'analyse (13) dispersée et disposé de telle sorte que de la lumière de longueurs d'onde différentes est incidente à différents endroits du réseau de capteurs (11), le dispositif (1) présentant un boîtier (16) et étant réalisé sous la forme d'une unité modulaire compacte qui contient au moins la source lumineuse (20 ; 20a, 20b), les moyens de dispersion (9) et le réseau de capteurs (11), et qui mesure moins de 20 mm x 30 mm x 100 mm, de préférence environ 10 mm x 15 mm x 50 mm, le boîtier étant de plus muni de moyens de fixation de telle sorte que le dispositif peut être fixé directement à un patient, le réseau de capteurs (11) bidimensionnel étant disposé de telle sorte que la lumière d'analyse dispersée est incidente sur une pluralité de rangées adjacentes du réseau de capteurs (11) bidimensionnel, dans lequel, en raison de la dispersion en fonction de la longueur d'onde de la lumière de mesure, la lumière de mesure est reproduite sur une rangée du capteur et le spectre est détecté en même temps par plusieurs rangées adjacentes en parallèle du réseau de capteurs,
le dispositif (1) présentant un système informatique qui est réalisé pour additionner les spectres générés par les rangées adjacentes,
le dispositif (1) présentant un diaphragme à fente (7) qui est disposé entre une zone d'entrée pour la lumière d'analyse (4) et les moyens (9) de dispersion, et le diaphragme à fente (7) est disposé par rapport aux moyens (9) de dispersion de la lumière de telle sorte qu'une image allongée est dispersée dans une direction différente de l'image allongée, de préférence perpendiculaire à celle-ci,
et le réseau de capteurs bidimensionnel (11) étant un capteur d'image CMOS ayant une fréquence d'image supérieure à 100 Hz.

**2.** Dispositif selon la revendication 1, **caractérisé en ce que** le boîtier (16) est réalisé pour être rattaché à un endroit du corps d'un patient humain, en particulier à un doigt ou à un lobe d'oreille.

**3.** Dispositif selon l'une quelconque des revendications 1 et 2, **caractérisé en ce que** les moyens de dispersion présentent un réseau de diffraction (9), en particulier un réseau holographique.

**4.** Dispositif selon l'une quelconque des revendications 1 à 3, **caractérisé en ce que** le dispositif présente un convertisseur A/N.

**5.** Dispositif selon l'une quelconque des revendications 1 à 4, **caractérisé en ce que** le dispositif présente un amplificateur qui est de préférence paramétrable.

**6.** Dispositif selon l'une quelconque des revendications 1 à 5, **caractérisé en ce que** le dispositif présente une connexion pour une liaison de communication électrique (29), et **en ce que** le dispositif ne présente en particulier aucune connexion pour l'arrivée ou l'évacuation de lumière.

**7.** Dispositif selon l'une quelconque des revendications 1 à 6, **caractérisé en ce que** le dispositif est réalisé pour la mesure de transmission dans laquelle la lumière d'analyse ressort à un autre endroit que la zone de mesure après transmission à travers les tissus, et pour la mesure de réflexion dans laquelle la lumière d'analyse est la lumière réfléchie directement par la zone de mesure.

8. Dispositif selon la revendication 7, **caractérisé en ce que** le dispositif présente un système informatique (26) qui est réalisé de telle sorte qu'une mesure de transmission et une mesure de réflexion peuvent être réalisées en alternance, le dispositif présentant une première source lumineuse pour effectuer une mesure de réflexion en éclairant une première zone de mesure (3') et une deuxième source lumineuse (20b) pour éclairer une deuxième zone de mesure (3") pour la mesure de transmission.

9. Dispositif selon la revendication 8, **caractérisé en ce que** le dispositif est équipé de moyens (17) pour séparer la lumière d'analyse (4) provenant d'une zone de réflexion (3') et d'une zone de transmission (3").

10. Dispositif selon l'une quelconque des revendications 1 à 9, **caractérisé en ce que** le dispositif est réalisé pour le balayage à une fréquence >50 Hz, une partie tissulaire et une partie pulsatile des valeurs sanguines physiologiques mesurées pouvant être déterminées.

11. Dispositif selon l'une quelconque des revendications 1 à 10, **caractérisé en ce que** le dispositif présente un système informatique (26) qui est réalisé de telle sorte que des mesures sont effectuées de manière résolue dans le temps.

12. Dispositif selon l'une quelconque des revendications 1 à 11, **caractérisé en ce que** le dispositif présente un système informatique (26) qui est réalisé de telle sorte qu'une dérivée seconde des spectres détectés est déterminée et que des valeurs sanguines physiologiques sont déterminées sur la base de cette dérivée seconde.

13. Combinaison d'un système informatique externe (32) et d'un dispositif selon l'une quelconque des revendications 1 à 10, dans laquelle le dispositif (1) et le système informatique (32) sont ou peuvent être reliés l'un à l'autre par un câble de communication (29), le système informatique étant réalisé de telle sorte que des mesures sont effectuées de manière résolue dans le temps, ou le système informatique étant réalisé de telle sorte qu'une dérivée seconde des spectres détectés est déterminée et que des valeurs sanguines physiologiques sont déterminées sur la base de cette dérivée seconde.

14. Procédé permettant d'identifier et de surveiller des valeurs sanguines physiologiques d'un être vivant lors d'une mesure in vivo non invasive, composé des étapes suivantes, avec un dispositif selon l'une quelconque des revendications 1 à 12,

   - appliquer au moins à la zone de mesure (3 ; 3', 3") de la lumière (2 ; 2a, 2b) provenant de la source lumineuse à large bande (20 ; 20a, 20b) pour générer de la lumière à large bande (2 ; 2a, 2b), comprenant de préférence au moins de 500 nm à 850 nm et/ou de 800 nm à 1200 nm, en particulier une LED,
   - détecter par le réseau de capteurs CMOS (11) une lumière d'analyse (4) renvoyée en réflexion et/ou en transmission, dans lequel
   - disperser en fonction de l'onde la lumière d'analyse détectée (4) par les moyens de dispersion et de reproduction des parties individuelles, dépendantes de la longueur d'onde, de la lumière d'analyse (4) détectée sur le réseau de capteurs CMOS (11) bidimensionnel, la lumière d'analyse dispersée, notamment le spectre, étant reproduite sur une pluralité de rangées adjacentes du réseau de capteurs (11) et les spectres générés par les rangées individuelles étant additionnés,
   - évaluer le spectre ainsi généré au moyen du système informatique qui est réalisé pour additionner les spectres générés par les rangées adjacentes afin de déterminer les valeurs sanguines,

   dans lequel au préalable, le boîtier est fixé à un point de mesure (3 ; 3', 3") d'un être vivant avec les moyens de fixation, lequel boîtier contient la source lumineuse et le réseau de capteurs.

15. Procédé selon la revendication 14, **caractérisé en ce que** la lumière d'analyse (4) est dispersée au niveau d'un réseau de diffraction (9).

16. Procédé selon la revendication 14 ou 15, **caractérisé en ce que** la lumière d'analyse (4) est détectée en réflexion, la lumière d'analyse étant la lumière réfléchie directement par la zone de mesure, et en transmission, la lumière d'analyse étant la lumière d'analyse ressortant à un autre endroit après transmission à travers les tissus.

17. Procédé selon l'une quelconque des revendications 14 à 16, **caractérisé en ce que** la lumière d'analyse (4) est évaluée de manière résolue dans le temps au moyen du système informatique.

18. Procédé selon l'une quelconque des revendications 14 à 17, **caractérisé en ce que** pour la détermination des valeurs sanguines physiologiques, la dérivée seconde des spectres détectés est déterminée dans un système informatique.

19. Procédé selon l'une quelconque des revendications 14 à 18, **caractérisé en ce qu'**un balayage est effectué à une fréquence supérieure à 50 Hz, et **en ce qu'**une partie tissulaire et une partie pulsatile des valeurs sanguines physiologiques mesurées sont déterminées au moyen du système informatique.

Fig. 1. a)

Fig. 1. b)

**Fig. 2**

3''

17

3'

15

**Fig. 3. a)**

17

15

3'        3''

**Fig. 3 b)**

**Fig. 4**

**Fig. 5**

Fig. 6 a)

Fig. 6 b)

**Fig. 7**

**Fig. 8 (1)**

ERSATZBLATT (REGEL 26)

34

**Fig. 8(2)**

FIG.9

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008132205 A1
- WO 2006094169 A1
- US 5086229 A
- US 5070874 A
- US 5360004 A
- EP 522674 A2
- US 20060167348 A
- WO 2009043554 A
- WO 03071939 A1
- US 5879294 A
- WO 2007048989 A1
- US 5931779 A
- US 5361758 A
- US 2007216898 A1
- US 6315955 B
- US 2005154277 A1
- US 6049727 A
- WO 9316629 A1
- US 2008208020 A1
- US 2005267346 A1
- US 6078828 A
- US 2003071216 A1
- WO 2009139315 A1
- DE 19518511

**In der Beschreibung aufgeführte Nicht-Patentliteratur**

- Enhancing calibration models for non-invasive near-infrared spectroscopical blood glucose determination. **CH. FISCHBACHER ; K.-U. JAGEMANN ; K. DANZER ; U. A. MÜLLER ; L. PAPENKORDT ; J. SCHÜLER.** Fresenius J Anal Chem. Springer-Verlag, 1997, vol. 359, 78-82
- **WOLFGANG SCHRADER ; PETRA MEUER ; JÜRGEN POPP ; WOLFGANG KIEFER ; JOHANNES-ULRICH MENZEBACH ; BERNHARD.** Non-invasive glucose determination in the human eye. *SchraderJournal of Molecular Structure,* 14. Februar 2005, vol. 735-736, 299-306
- **PETRA MEUER.** Dissertation. Universität Würzburg, 2002
- **DAMIANOU, D. ; CROWE, J.A.** The wavelength dependence of pulse oximetry. *Pulse Oximetry: A Critical Appraisal, IEE Colloquium,* 29. Mai 1996, vol. 1996 (124), 7, , 1-7, 3
- **MANNHEIMER PH.D.** The light-tissue interaction of pulse oximetry. *Anesth. Analg.,* Dezember 2007, vol. 105 (6), 10-7
- LED Based Sensor System for Non-Invasive Measurement of the Hemoglobin Concentration in Human Blood. **U. TIMM ; E. LEWIS ; D. MCGRATH ; J. KRAITL ; H. EWALD.** 13th International Conference on Biomedical Engineering. Springer, 2009, vol. 23